(19) Europäisches Patentamt
European Patent Office
Office européen des brevets

(11) **EP 3 136 989 B1**

(12) **EUROPÄISCHE PATENTSCHRIFT**

(45) Veröffentlichungstag und Bekanntmachung des Hinweises auf die Patenterteilung:
**25.09.2019 Patentblatt 2019/39**

(21) Anmeldenummer: **15718228.8**

(22) Anmeldetag: **24.04.2015**

(51) Int Cl.:
***A61B 17/128*** (2006.01)   ***A61B 17/122*** (2006.01)

(86) Internationale Anmeldenummer:
**PCT/EP2015/058944**

(87) Internationale Veröffentlichungsnummer:
**WO 2015/165820 (05.11.2015 Gazette 2015/44)**

(54) **CHIRURGISCHER CLIP**

SURGICAL CLIP

CLIP CHIRURGICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2014 DE 102014207955**

(43) Veröffentlichungstag der Anmeldung:
**08.03.2017 Patentblatt 2017/10**

(73) Patentinhaber: **Aesculap AG**
**78532 Tuttlingen (DE)**

(72) Erfinder:
- **SCHOLTEN, Thomas**
 **78532 Tuttlingen (DE)**
- **WANKE, Gunnar**
 **8280 Kreuzlingen (CH)**
- **DROST, Erick**
 **78727 Oberndorf a. N. (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**EP-A1- 1 810 622      EP-A1- 2 389 878**
**EP-A2- 0 567 965      EP-A2- 0 576 835**
**WO-A2-2012/135141      DE-A1-102009 018 820**
**US-A1- 2013 072 947**

- **Erich Krabbe: "Stanztechnik: Erster Teil, Schnittechnik, Technologie des Schneidens" In: "Stanztechnik: Erster Teil, Schnittechnik, Technologie des Schneidens", 1. Januar 1940 (1940-01-01), Springer-Verlag GmbH, XP055254653, ISBN: 978-3-662-37376-7 Seiten 11-11, DOI: 10.1007/978-3-662-38122-9,**

EP 3 136 989 B1

**Beschreibung**

Die vorliegende Erfindung betrifft einen chirurgischen Clip. Insbesondere betrifft diese Erfindung einen chirurgischen Clip, welcher im verpressten Zustand, d. h. dann, wenn Gewebe zwischen den Cliparmen zusammengehalten bzw. gedrückt wird, einen vorteilhaften Verlauf der Verpresskraft entlang der Cliparme aufweist.

Stand der Technik

Die EP 2 389 878 A1, EP 1 810 662 A1 und EP 0 576 835 A2 offenbaren Clipgeometrien, die der Präambel des unabhängigen Anspruches 1 entsprechen. Im Stand der Technik sind einige chirurgische Clips bekannt. Die Firma Applied Medical vertreibt einen chirurgischen Clip EPIX Universal CA500, bei dem die beiden im Wesentlichen geraden Cliparme über einen im Wesentlichen V-förmigen Abschnitt miteinander verbunden sind. Die beiden geraden Cliparme verlaufen im Wesentlichen parallel zur Längsachse des chirurgischen Clips und in den Übergangsbereichen zwischen den Cliparmen und dem Verbindungsabschnitt sowie an der Kehle des Verbindungsabschnitts sind relativ kleine Biegeradien ausgebildet. Dies bedeutet, dass die Übergangsbereiche durch Knicke gebildet sind. Vergleichbare chirurgische Clips mit vergleichbarer Geometrie werden auch von United States Surgical unter dem Namen Endo Clip Autosuture 5 mm und von Ethicon unter dem Namen Ligamax 5 vertrieben.

Eine etwas andere Geometrie weist der Endo Clip Autosuture III 5 mm auf, der ebenfalls von United States Surgical vertrieben wird. Dieser Clip weist ebenfalls zwei im Wesentlichen gerade, parallele Cliparme sowie einen Verbindungsabschnitt für die beiden geraden Cliparme auf, der mit einer Kehle ausgebildet ist, welche einen recht kleinen Krümmungsradius aufweist. Im Gegensatz zu den vorbeschriebenen chirurgischen Clips sind die Übergangsbereiche zwischen den Cliparmen und dem Verbindungsabschnitt mit einem deutlich größeren Krümmungsradius ausgebildet, also eher als gebogene Abschnitte denn als geknickte Abschnitte.

Die Patentanmeldung US 2011/0224701 A1 offenbart einen chirurgischen Clip mit einer halbkreisförmigen Außenfläche und einer profilierten Innenfläche. Die halbkreisförmigen Außenfläche dient dazu, ein Verkeilen des Clips im Maulteil des Clipapplikators zu verhindern und die profilierte Innenfläche soll die Haftung auf dem geklemmten Gewebe verbessern. Die Cliparme des Clips weisen in einer Seitenansicht jeweils gerade Abschnitte auf, welche im Bereich des distalen Endes des Clips, also an seiner offenen Seite, parallel zueinander verlaufen. Die Cliparme bestehen zudem aus mehreren im Wesentlichen unverformbaren Abschnitten, welche durch verformbare Abschnitte verbunden sind.

Allen bekannten Clips ist gemeinsam, dass jeder Cliparm einen im Wesentlichen geraden Abschnitt aufweist und der Verbindungsabschnitt zwei im Wesentlichen gerade Abschnitte aufweist. In der Kehle sowie im Übergangsbereich zwischen Cliparm und Verbindungsabschnitt sind dann mehr oder weniger gekrümmte Abschnitte ausgebildet. All diese Clips sind zudem Einfachstegclips, d.h. Clips, die aus einem Stück Draht gebogen werden können und sich im Wesentlichen in einer Ebene erstrecken (abgesehen von der Drahtsicke). Einfachstegclips weisen immer zwei Cliparme auf.

Ein Problem dieser Art von Clips ist, dass sie beim Applizieren, d.h. beim Verpressen durch einen Clipapplikator, ungünstige Eigenschaften offenbaren. Zum einen wird durch die Ausbildung des recht geringen Krümmungsradius im Übergangsbereich von Cliparm zu Verbindungsabschnitt ein Bereich geschaffen, in dem das Material des Clips (Metall, beispielsweise Titan oder Titanlegierungen) mehr gestreckt wird als in den angrenzenden Bereichen, was dazu führt, dass dieser Bereich, im Folgenden Knick genannt, beim Verpressen des Clips mit einem Applikator nicht vollständig in eine gerade Form zurückverformt werden kann. Es verbleibt somit ein Stelle in dem verpressten Clip, an dem die beiden Cliparme weiter beabstandet sind. Dies führt zu einem nicht optimalen Verschluss des jeweils geclipsten, d.h. zusammengedrückten Gefäßes.

Ein weiteres Problem dieser Art von Clips ist, dass der Verschluss dieser Clips im verpressten Zustand an den distalen Enden der Cliparme schwach ist. Dies bedeutet, dass an den distalen Clipenden kaum mehr eine Kraft auf das Gefäß aufgebracht wird, welche zu einem Verschluss des Gefäßes führt. Während des Verpressvorgangs werden die beiden parallelen Cliparme um die Kehle des Clips herum nach innen verformt. Dabei verbleibt nur der Knick bzw. der Übergangsbereich des Clips in Kontakt mit der jeweiligen Branche des Clipapplikators. Erst wenn die distalen Enden des Clips sich berühren (falls kein Gewebe gegriffen wird) oder beiderseits des zu greifenden Gewebes in Anlage kommen (wenn Gewebe gegriffen wird), wird der Knick zwischen Cliparm und Verbindungsabschnitt aufgebogen. Dabei verformen sich die distalen Enden der Cliparme nach außen und der maßgebliche Krafteinleitungspunkt in das Gewebe verlagert sich zu dem Knick hin. Dies entlastet wiederum die distalen Enden der Cliparme und sie entspannen ihre elastische Verformung (im Wesentlichen unter Beibehaltung ihrer aktuellen Lage). Wenn nun der Knick in dem Cliparm weitest möglich zurückverformt wurde, der Clip also vollständig verpresst ist, führt dies dazu, dass der Clip an den distalen Enden der Cliparme kaum mehr eine Schließkraft bzw. Verpresskraft aufbringen kann, da sich die distalen Enden der Cliparme leicht elastisch nach außen verformen lassen und da es im mittleren Bereich des Clips Stellen gibt, an denen sich die Cliparme berühren (falls keine Gewebe gegriffen wird) oder die Cliparme beiderseits des gegriffenen Gewebes im Wesentlichen punktuell anliegen (dieser Bereich ist im Folgenden in beiden Fällen als mittlerer Kontaktbereich bezeichnet). Auf diese Weise sind die distalen Enden nämlich gegen Ende des Verpressvorgangs im Wesentlichen vollständig entlastet worden, als der Knick in dem Cliparm mit einer Kraft zurückverformt wurde, die jene Kraft bei weitem überschreitet, welche erforderlich ist, um die übrigen Bereiche des Clips in die verpresste Form des Clips zu bringen. Die Verpresskraft, die eigentlich möglichst gleichmäßig über die Länge des verpressten Clips verteilt sein sollte, konzentriert sich dann auf die Nähe der Clipkehle sowie den mittleren Kontaktbereich des Clips.

Aufgabe der Erfindung

Die Aufgabe der vorliegenden Erfindung ist es, einen chirurgischen Clip zu schaffen, bei dem sich ein gleichmäßiger und möglichst kleiner Spalt zwischen den Cliparmen bildet und bei dem die Cliparme bis zu ihrem distalen Ende hin eine ausreichende Verpresskraft liefern. Eine weitere Aufgabe der vorliegenden Erfindung ist es, einen chirurgischen Clip bereit zu stellen, welcher über im Wesentlichen die gesamte Cliplänge eine gleichmäßige Kraft auf das gegriffene Gewebe ausübt. Noch eine weitere Aufgabe der vorliegenden Erfindung ist es, ein Herstellungsverfahren für einen solchen Clip bereit zu stellen.

Begriffsdefinitionen

Ein Clip hat einen unverpressten Ausgangszustand und einen verpressten Endzustand. Normalerweise befindet sich im verpressten Zustand Gewebe zwischen zwei zusammen gehörende Cliparme, beispielsweise ein Hohlorgan wie ein Blutgefäß. In diesem Fall bestimmen die Dicke und die Konsistenz der Wandung des Blutgefäßes die Breite des Spalts zwischen den Cliparmen im verpressten Zustand. Aus diesem Grund beziehen sich Angaben zur Spaltdicke des Clips im verpressten Zustand in diesen Ausführungen immer auf den Fall, dass kein Gewebe zwischen den Cliparmen befindlich ist, da nur so ein sinnvoller Vergleich mit den Clips aus dem Stand der Technik möglich ist. Dazu kann ausgeführt werden, dass die Qualität eines Spaltes des Clips im verpressten Zustand ohne dazwischen liegendem Gewebe ein klarer Hinweis auf die Qualität des Spaltes mit dazwischen liegendem Gewebe gibt. Die Qualität des Spaltes bestimmt sich auch Spaltbreite und durch die Cliparme aufgebrachter Verpresskraft entlang der Cliparme. Bildet sich zum Beispiel bei einem Clip ohne dazwischen liegendem Gewebe ein paralleler Spalt aus, der im Wesentlichen über seine gesamte Länge eine im Wesentlichen gleich große Verpresskraft aufweist, so würde sich bei demselben Clip mit dazwischen liegendem Gewebe im Wesentlichen über die gesamte Länge der Gewebe-Clip-Kontaktlinie/-fläche eine ebenso im Wesentlichen gleich große Verpresskraft ergeben.

Ein Clip besteht aus einer geraden Anzahl von Cliparmen, von denen je zwei einander zugeordnet sind und eine Ebene bilden. Bei mehreren Cliparmpaaren werden so mehrere im Wesentlichen parallele Ebenen gebildet. Die Clipebene ist eine Ebene, die parallel zu den vorstehend beschriebenen Ebenen verläuft und die den Clip in der Richtung senkrecht zu dieser Ebene im Wesentlichen halbiert. Jeweils ein Paar von zusammengehörigen Cliparmen ist an seinen proximalen Enden miteinander verbunden und bildet im Verbindungsbereich eine Clipkehle aus. Die Längsachse eines Clips oder eines Paares von Cliparmen ist eine Linie von der Clipkehle zu der Mitte zwischen den beiden distalen Enden der jeweiligen Cliparme. Diese Längsachse (strichpunktierte Linie) verläuft proximal-distal.

Die Cliplänge L ist die Entfernung von der Außenseite der Clipkehle zu der senkrechten Projektion der distalen Enden der Cliparme auf die Längsachse des Clips, die Clipbreite B ist der Abstand der Außenseiten der distalen Enden zweier zusammen gehöriger Cliparme (d.h. diese beiden Cliparme bilden zusammen ein Paar von Cliparmen) und die Cliphöhe H ist die Erstreckung des Clips in einer Richtung senkrecht zur Clipebene. Die Cliplänge L, die Clipbreite B und die Cliphöhe H beziehen sich auf die Außenmaße des jeweiligen Clips.

Die lichte Cliplänge $L_I$ ist die Entfernung von der Innenseite der Clipkehle zu der senkrechten Projektion der distalen Enden der Cliparme auf die Längsachse des Clips, die lichte Clipbreite $B_I$ ist der Abstand der Innenseiten der distalen Enden zweier zusammen gehöriger Cliparme (d.h. diese beiden Cliparme bilden zusammen ein Paar von Cliparmen) und die lichte Cliphöhe $H_I$ ist der Abstand zweier benachbarter Cliparme, welche zusammen kein Paar von zusammen gehörigen Cliparmen bildet. Die lichte Cliplänge $L_I$, die lichte Clipbreite $B_I$ und die lichte Cliphöhe $H_I$ beziehen sich auf die Innenmaße des jeweiligen Clips.

Die Cliparmlänge l ist die Länge der Abwicklung eines Cliparms in eine Gerade, wobei sich die Cliparmlänge l auf die neutrale Faser (gestrichelte Linie) des Cliparms bezieht. Die neutrale Faser ist der geometrische Ort der Zentren der Flächenträgheitsmomente entlang des jeweiligen Cliparms. Die Länge auf Cliparm $l_a$ wird von der Clipkehle aus in Richtung distalem Ende des Cliparms gemessen. Die Länge auf Cliparm $l_a$ wird in Prozent (%) angegeben, wobei eine Länge auf Cliparm von 0% der Clipkehle entspricht, also dem Ort, an dem die neutrale Faser des Cliparms bzw. zweier zusammen gehöriger Cliparme die Längsachse des Clips schneidet, und eine Länge auf Cliparm von 100% dem distalen Ende des jeweiligen Cliparms entspricht. Die Cliparmbreite b entspricht bei einem aus einem Blech hergestellten Ringclip dem Abstand der seitlichen Kanten eines Cliparms. Die Cliparmhöhe h entspricht bei einem aus einem Blech hergestellten Ringclip der Dicke des Blechs. Wenn die Cliparmbreite und/oder die Cliparmhöhe über die Cliparme veränderlich ist, können verschiedene bzw. veränderliche Cliparmbreiten und -höhen b', b'', h' vorliegen. Der Tangentenwinkel a ist der Winkel, den eine Tangente an die neutrale Faser des jeweiligen Cliparms mit einer Geraden einnimmt, welche senkrecht auf die Cliplängsachse steht. Der Tangentenwinkel $\alpha$ liegt somit immer

zwischen 0° und im Wesentlichen 90°.

Wenn nichts anderes gesagt wird, bezieht sich die Beschreibung des Clips im Folgenden auf den unverpressten Zustand des Clips, in dem dieser spannungsfrei ist.

Der Begriff des chirurgischen Rohrschaft-Instruments umfasst in dieser Anmeldung zum einen endoskopische Instrumente wie beispielsweise endoskopische Clipapplikatoren. Zum anderen umfasst dieser Begriff aber auch chirurgische Instrumente für eine offene Operation, bei denen der Funktionsabschnitt bzw. der Wirkabschnitt des Instruments durch einen Schaft oder ein schaftartiges Bauteil von dem Betätigungsabschnitt bzw. dem Griffabschnitt getrennt ist. Der Begriff Schaft bzw. schaftartiges Bauteil bezeichnet hierbei ein Bauteil, dessen Abmessungen und Lage gegenüber dem Betätigungsabschnitt (z.B. Griffstück) auch während einer Betätigung des chirurgischen Instruments im Wesentlichen unveränderlich ist. Eine axiale Verschiebung entlang der Achse des Schafts oder schaftartigen Bauteils bzw. eine Verdrehung um diese Achse herum ist dabei zulässig, nicht jedoch eine wesentliche Verschiebung quer zu dieser Achse oder eine Verdrehung gegenüber dieser Achse in der Weise, dass sich die beiden Enden des Bauteils wesentlich von dieser Achse entfernen. Vorzugsweise ist die Länge eines Schafts oder schaftartigen Bauteils größer als dessen beide anderen Abmessungen (Breite, Tiefe) und es ist weiter vorzugsweise schlank ausgebildet. Der Schaft bzw. das schaftartige Bauteil muss dabei nicht rund, geschlossen, rohrförmig oder dünnwandig sein. Entscheidend ist hierbei, dass es sich um ein Instrument handelt, welches nicht wie eine gewöhnliche Schere einen Drehpunkt aufweist, um welchen sich alle wesentlichen Bestandteile des Instruments drehen, sondern dass die Kraft zum Öffnen und Schließen des Maulteils über eine relative axiale Bewegung eines Bauteils gegenüber dem Schaft übertragen wird.

Der Funktionsabschnitt bzw. Wirkabschnitt ist in dieser Anmeldung der Bereich des chirurgischen Rohrschaft-Instruments, an dem dessen eigentliche Funktion ausgeführt wird. Bei einem Nadelhalter ist es der Bereich, der die Nadel greift und hält, also die distalen Bereiche der Branchen. Bei einer Schere ist es der Bereich, der das Gewebe oder etwas anderes durchtrennt, also der Bereich, an dem die beiden aneinander vorbei gleitenden Scherkanten ausgebildet sind. Bei einem Clipapplikator ist es der Bereich, in dem der Clip zunächst gehalten wird, während er durch den Chirurgen an die richtige Stelle und in die richtige Lage gebracht wird, und in dem der Clip anschließend appliziert wird, d.h. verpresst wird. Bei anderen Instrumenten ist die Definition des Funktionsabschnitts bzw. Wirkabschnitts entsprechend anzuwenden.

Der Wirkbereich ist der Bereich einer einzelnen Branche, an dem diese die bestimmungsgemäße Funktion des Instruments bewirkt, also bei einem Nadelhalter ein Greifbereich, bei einer Schere eine Scherkante und bei einem Clipapplikator ein Anlagebereich des Clips.

Ein Schließen bzw. ein Schließvorgang des Maulteils bedeutet hier, dass sich die Wirkbereiche der Branchen während des Schließvorgangs vorwiegend aufeinander zu bewegen. Im Falle einer Schere bewegen sich die Wirkbereiche der Branchen, also die Scherkanten, im späteren Verlauf des Schließvorgangs aneinander vorbei und dadurch wieder auseinander, trotzdem wird dieser ganze Vorgang als Schließvorgang des Maulteils bezeichnet. Allgemein gesagt bezeichnet ein Schließvorgang die Durchführung der dem Instrument zugewiesenen Funktion wie beispielsweise ein Greifen von Gewebe oder bspw. einer Nadel, ein Schneiden von Gewebe oder anderen Gegenständen, ein Applizieren eines Clips oder ein Aufspreizen von Gewebe oder anderen Gegenständen wie bspw. einem Clip. Ein sich anschließendes Öffnen bzw. ein sich anschließender Öffnungsvorgang bezeichnet dann allgemein die Rückkehr der Branchen, also auch der Wirkbereiche bzw. der distalen Bereiche der Branchen des Maulteils des jeweiligen Instruments, in ihre Ausgangsposition. Bei einem chirurgischen Spreizer ist die Zuordnung genau anders herum, da er seine Aufgabe ausübt, wenn sich die Branchen im Wesentlichen voneinander entfernen, also einen Öffnungsvorgang durchführen, wohingegen ein Zurückkehren der Branchen in die Ausgangsposition einem Schließvorgang entspricht. Trotzdem gibt es auch bei einem solchen Instrument einen klaren Öffnungsvorgang und einen klaren Schließvorgang.

Allgemeine Beschreibung der Erfindung

Die Aufgabe der vorliegenden Erfindung wird gelöst durch einen Clip nach Anspruch 1 bzw. ein Herstellungsverfahren nach einem der Ansprüche 16 oder 17. Vorteilhafte Ausgestaltungen des chirurgischen Clips bzw. der Herstellungsverfahren sind Gegenstand der abhängigen Ansprüche.

Gemäß einer Ausführungsform der vorliegenden Erfindung ist ein chirurgischer Clip für einen chirurgischen Clipapplikator offenbart mit wenigstens einem Paar von Cliparmen, wobei jeder Cliparm ein distales Ende und ein proximales Ende aufweist. Die beiden Cliparme eines Paares von Cliparmen sind an ihren proximalen Enden miteinander verbunden, sodass sie eine Clipkehle ausbilden. Üblicherweise sind die Cliparme eines Paares von Cliparmen aus einem Stück gefertigt und somit stofflich verbunden. Die Cliparme können prinzipiell aber auch einzeln ausgebildet sein und anschließend beispielsweise durch Schweißen miteinander verbunden werden. Ein Tangentenwinkel zwischen einer Tangente an eine neutrale Faser eines Cliparms und einer Senkrechten auf eine Längsachse des Clips wächst im Wesentlichen über die gesamte Länge des Cliparms stetig. Die bedeutet, dass jeder Cliparm eine durchgehend gebogene Form aufweist. Es kommt hierbei nicht darauf an, dass es gar keine geraden Abschnitte auf einem Cliparm gibt, da sich ein Bogen auch durch einen entsprechend fein gegliederten Polygonzug annähern lässt. Es ist aber Wesentlich, dass keine klar erkennbare Folge von geraden Abschnitten und Knickstellen vorhanden sind.

Ein solcher chirurgischer Clip mit einer durchgehenden Bogenform hat den Vorteil, dass nach dem Verpressen eine gleichmäßigere und höhere Kraftaufbringung auf das durch den Clip gegriffene Gewebe erfolgt als dies mit den Clips aus dem Stand der Technik möglich ist. Besteht ein chirurgischer Clip aus einer Folge von geraden Abschnitten und Knickstellen, so wie dies häufig im Stand der Technik der Fall ist, führt dies zu einer ungleichmäßigen Verteilung der auf das gegriffene Gewebe aufgebrachten Kraft. Dies liegt daran, dass an den Knickstellen mehr Kraft von dem Clipapplikator in den Clip eingeleitet wird als in deren Umgebung, da diese Knickstellen die Hauptkontaktstellen mit der Innenfläche des Mauls des Clipapplikators bilden. Bei den Clips Endo Clip autosuture 5 mm, Endo Clip autosuture III 5 mm und EPIX Universal CA500 wird über den gesamten Verlauf des Zusammenpressens des Clips fast ausschließlich an den Außenseiten der Knickstellen Kraft von dem Clipapplikator in den Clip eingeleitet. Lediglich ganz am Anfang des Verpressvorgangs wird eine Kraft entlang der Cliparme eingeleitet, sobald aber die Clipkehle verformt wird, lösen sich die distalen Enden der Clips vom Clipapplikator. Zudem sind die Knickstellen aufgrund der kalten plastischen Verformung bei deren Ausbildung steifer als andere Abschnitte des Clips. um diese Knickstellen wieder zu einem geraden Abschnitt zurück zu verformen, was erforderlich ist, um einen gleichmäßigen Clipspalt zu erhalten, muss übermäßig viel Kraft in den Clip eingeleitet werden. Dabei kommen die distalen enden des Clips mit dem Gewebe frühzeitig in Kontakt und werden dadurch nach außen verformt. Teilweise erfolgt dabei sogar eine plastische Verformung nach außen, was keinesfalls gewünscht ist und sich sehr negativ auf die Verschlusskraft des Clips im distalen Bereich auswirkt. Mit der erfindungsgemäßen Clipform erfolgt, wenn überhaupt, nur eine elastische Verformung der distalen Clipenden nach außen, sodass die maximal mögliche Verschlusskraft auch im distalen Bereich erhalten bleibt. Zudem müssen keine Knickstellen zurückverformt werden.

Gemäß einer vorteilhaften Ausführungsform der vorliegenden Erfindung weist der Tangentenwinkel bei einer Länge auf Cliparm von 6% einen Wert von höchstens 40° auf, vorzugsweise einen Wert von vorzugsweise höchstens 30°, und weiter vorzugsweise einen Wert von 20° bis 30°. Ein chirurgischer Clip unterliegt verschiedensten Randbedingungen. Zum Einen soll er eine möglichst große Öffnungsweite aufweisen, sodass auch dickes Gewebe oder ein dickes Gefäß gegriffen werden kann. Dazu muss er eine entsprechende Öffnungsweite aber nicht nur am distalen Ende aufweisen, sondern auch in seinem proximalen Bereich, sodass das Gewebe bzw. das Gefäß vor einem Verpressen auch vollständig bzw. soweit wie erforderlich in dem Clip aufgenommen werden kann. Zum Anderen soll der Clip aber auch so klein wie möglich sein, sodass er bei einer endoskopischen Applikation mit möglichst dünnen endoskopischen Applikatoren appliziert werden kann. Die Cliparme sollen möglichst steif sein, um das gegriffene Gewebe sicher zusammenzuhalten bzw. ein gegriffenes Gefäß sicher abzuklemmen. Andererseits sollen die Arme nicht unnötig steif sein, da dies wiederum steifere Bauteile im Applikator erfordert, was dem Wunsch nach immer schlankeren Applikatoren entgegen steht. Damit der Clip eine Form erhält, die eine gleichmäßige und ausreichende Kraftaufbringung auf das zu greifende Gewebe ermöglicht, nimmt der Tangentenwinkel bei einer Länge auf Cliparm von 6% einen Winkel von höchstens 40°, vorzugsweise einen Wert von vorzugsweise höchstens 30°, und weiter vorzugsweise einen Wert von 20° bis 30° an. Bei größeren Winkeln an dieser Stelle auf dem Cliparm müssten sonst knickähnliche Bereiche entlang des Cliparms ausgebildet werden, um ein vollständiges Schließen des Clips zu ermöglichen. Solche knickähnlichen Bereiche aber führen zu den oben beschriebenen Problemen bzgl. der ungleichmäßigen Kraftaufbringung auf das zu greifende Gewebe. Damit eine hinreichend große Öffnung in dem Clip gebildet wird, um das zu greifende Gewebe aufnehmen zu können, bevor der Clip verpresst wird, soll dieser Winkel bei 6% Länge auf Cliparm nicht größer sein. Bei größeren Tangentenwinkeln an dieser Stelle kann nicht immer sicher gestellt werden, dass das zu greifende Gewebe in den Clip eingebracht werden kann. Insbesondere wenn sich der Tangentenwinkel in einem Bereich von 20° bis 30° befindet, kann das Gewebe gut in den Clip eingeführt werden bzw. der Clip auf das Gewebe aufgeschoben werden. Gleichzeitig kann eine gleichmäßige Verschlusskraft durch einen solchen Clip sicher gestellt werden.

Gemäß einer weiter vorteilhaften Ausführungsform der vorliegenden Erfindung weist der Tangentenwinkel bei einer Länge auf Cliparm von 15% einen Wert von mindestens 58° auf, vorzugsweise einen Wert von 58° bis 75°. Mit einem Tangentenwinkel von mindestens 60° an dieser Stelle kann sicher gestellt werden, dass die Krümmung des Cliparms über die verbleibende Länge auf Cliparm bis zum distalen Ende des Cliparms nicht zu groß ausgebildet werden muss, sodass sich Bereiche ausbilden würden, die Knickstellen ähneln und im verpressten Clip zu einem ungleichmäßigen Spalt führen würden. Mit einem Tangentenwinkel von maximal 75° an dieser Stelle kann sicher gestellt werden, dass auch distal von dieser Stelle noch eine hinreichende Krümmung in dem Cliparm vorgesehen werden kann, sodass dieser distale Bereich im verpressten Zustand elastisch nach lateral verformt wird, um eine hinreichende Kraft auf das gegriffene Gewebe auszuüben.

Gemäß der vorliegenden Erfindung weist der Tangentenwinkel bei einer Länge auf Cliparm ($l_a$) von 22% einen Wert von mindestens 60° aufweist, vorzugsweise einen Wert von mindestens 67° und weiter vorzugsweise einen Wert von 67° bis 80°. Mit dieser Beschränkung wird eine noch gleichmäßigere Kraftaufbringung auf das gegriffene Gewebe im verpressten Zustand des Clips erzielt.

Gemäß noch einer vorteilhaften Ausführungsform der vorliegenden Erfindung weist der Tangentenwinkel bei einer Länge von 27% auf Cliparm von einen Wert von mindestens 65° auf, vorzugsweise einen Wert von mindestens 72°, und weiter vorzugsweise einen Wert von 72° bis 85°. Mit dieser Beschränkung wird eine noch gleichmäßigere Kraftaufbringung auf das gegriffene Gewebe im verpressten Zustand des Clips erzielt.

Gemäß noch einer weiter vorteilhaften Ausführungsform der vorliegenden Erfindung weist der Tangentenwinkel bei einer Länge auf Cliparm von ca. 0% einen Wert von höchstens 5° aufweist, vorzugsweise einen Wert von im Wesentlichen 0°. Dies bedeutet, dass die Clipkehle keinen oder nur einen sehr geringen Knick ausbildet. Dies unterscheidet den erfindungsgemäßen Clip von fast allen bisherigen chirurgischen Clips und sorgt dafür, dass die Clipkehle beim Verpressen stark plastisch verformt wird. Aus dieser plastischen Verformung erhält der erfindungsgemäße Clip die Verpresskraft, die er über die gesamte Länge des Cliparms auf das gegriffene Gewebe aufbringt. Es ist klar, dass sich in der Praxis keine absoluten Knicke ausbilden werden, sondern immer gekrümmte Abschnitte entstehen. Die vorstehende Formulierung soll klar stellen, dass in der Clipkehle kein Knick ausgebildet ist, sondern dass der Clip die Clipachse im Bereich der Clipkehle im Wesentlichen senkrecht quert.

Gemäß einer weiter vorteilhaften Ausführungsform der vorliegenden Erfindung weist der Tangentenwinkel bei einer Länge auf Cliparm von 100% einen Wert von höchstens 88° auf, vorzugsweise einen Wert von 80° bis 88°. Diese Ausbildung führt dazu, dass der Clip im unverpressten Zustand mit seinen distalen Enden an dem Clipapplikator anliegt und dort zu Begin des Verpressens auch die Kraft eingeleitet wird. Der Krafteinleitungspunkt verlagert sich dann aufgrund der elastischen Verformung der Cliparme weiter nach proximal, ohne dass sich aber zu Begin des Verpressens die distalen Enden der Cliparme von dem Clipapplikator lösen. Dies bedeutet, dass die distalen Enden der Cliparme die lichte Öffnung des Clips nicht wie bei den Clips aus dem Stand der Technik gegenüber der lichten Öffnungsweite des Applikators verkleinern. Bei den Clips der bekannten Bauart mit einem Tangentenwinkel von 90° an den distalen Enden der Cliparme und wenigstens einer Knickstelle pro Clipbranche wird die Kraft im Wesentlichen an den Knickstellen vom Applikator in den Clip eingeleitet, die distalen Enden lösen bzw, entfernen sich dabei von den Branchen des Clipapplikators nach innen und sorgen dafür, dass der Abstand der distalen Enden der Cliparme deutlich kleiner als der Abstand der beiden Branchen des Clipapplikators wird. Dies kann dazu führen, dass ein leicht angepresster Clip ein Gewebestück nicht mehr aufnehmen kann, da sich dies an einem oder beiden distalen Enden des Clips der bekannten Bauart verhakt. Mit der vorstehenden Ausbildung wird sicher gestellt, dass auch ein leicht angepresster Clip noch das zu greifende Gewebe aufnehmen kann, ohne dieses zu beschädigen (beispielsweise mit dem relativ spitz ausgebildeten distalen Ende des Clips zu durchbohren).

Gemäß einer anderen vorteilhaften Ausführungsform der vorliegenden Erfindung ist der Cliparm in der Ebene des chirurgischen Clips von seinem distalen Ende bis zu seinem proximalen Ende so geformt, dass eine erste Ableitung der Krümmung der neutralen Faser des Cliparms im Bereich einer Länge auf Cliparm von 8% bis 100% keinen Vorzeichenwechsel aufweist. Dies bedeutet, dass die Form des Cliparms über diesen gesamten Bereich hinweg nicht von konvex zu konkav wechselt. Diese Ausbildung sorgt für eine besonders gleichmäßige Verteilung der Anpresskraft im verpressten Clip über die gesamte Cliplänge.

Gemäß wieder einer anderen vorteilhaften Ausführungsform der vorliegenden Erfindung sind wenigstens zwei Paare von Cliparmen vorgesehen, wobei ein Cliparm eines Paares von Cliparmen an seinem distalen Ende mit wenigstens einem distalen Ende eines anderen Cliparms eines anderen Paares von Cliparmen verbunden ist und einen distalen Verbindungsabschnitt bildet. Dies bedeutet, dass ein Doppelstegclip oder ein Mehrstegclip ausgebildet ist, bei welchem die einzelnen Cliparmpaare jeweils an den distalen Enden der Cliparme miteinander verbunden sind. Häufig werden solche Clips als Ringclips, Doppelringclips bzw. Mehrringclips ausgebildet, indem sie aus einem Blech z.B. ausgebildet werden, beispielsweise durch Ausstanzen oder Laserschneiden. Einzelstegclips, also Clips mit genau einem Paar von Cliparmen werden hingegen normalerweise aus einem Draht geformt, der auf eine bestimmte Länge abgelängt und anschließend gebogen wird. Es ist aber auch möglich, den Draht erst zu biegen und dann abzulängen.

Gemäß noch einer anderen vorteilhaften Ausführungsform der vorliegenden Erfindung weist der Clip zwei bzw. drei Paare von Cliparmen auf und der Clip ist als offener oder geschlossener Ringclip oder Doppelringclip ausgebildet, vorzugsweise mittels Stanzen, Lasersintern, Walzen, Gießen, Metal Inject Molding und/oder Schneiden, insbesondere Laserschneiden oder Wasserstrahlschneiden. Ein geschlossener Ringclip, Doppelringclip oder auch Mehrringclip ist ein Clip, bei dem zwei benachbarte Paare von Cliparmen jeweils einen geschlossenen Ring ausbilden. Dementsprechend ist ein offener Ringclip, Doppelringclip oder auch Mehrringclip ist ein Clip, bei dem zwei benachbarte Paare von Cliparmen jeweils einen Ring ausbilden, der an einer Stelle unterbrochen ist. Dies kann vorteilhaft sein, wenn man Gewebe verpressen möchte, welches im verpressten Zustand in nahe beieinander liegenden Bereichen eine deutlich unterschiedliche Dicke aufweist. Mit einem offenen Ringclip kann dann jedes Paar von Cliparmen soweit zusammengepresst werden, dass die optimale Verpressung des Gewebes erreicht wird.

Gemäß einer weiteren vorteilhaften Ausführungsform der vorliegenden Erfindung ist im Bereich des distalen Endes eines Cliparms und/oder eines distalen Verbindungsabschnitts eine Aussparung vorgesehen, die nach proximal hin offen und nach distal hin geschlossen ist, sodass die Aussparung von proximal zugänglich ist und eine erste Anlagefläche nach distal bildet und vorzugsweise eine zweite Anlagefläche nach lateral bildet. Die Aussparung ist dabei weiter vorzugsweise mittels Stanzen und/oder Prägen hergestellt. Mit einer ersten Anlagefläche am distalen Ende des Clips kann ein Zusammenwirken mit einem entsprechenden Vorsprung an einer Branche eines Clipapplikators erreicht werden, welche verhindert, dass ein Clip, wenn er auf das zu greifende Gewebe aufgeschoben wird, durch das Gewebe nach proximal aus dem Maul des Clipapplikators geschoben wird. Dies bedeutet, dass diese Anlagefläche dafür sorgt, dass der Clip an der korrekten Position im Maul eines Clipapplikators gehalten wird. Ansonsten kann es passieren, dass das zu greifende Gewebe den Clip gegenüber dem Maulteil des Clipapplikators nach proximal verschiebt, weil der Clip z.B. im Bereich der Clipkehle in Anlage an das Gewebe gelangt und der Clipapplikator vom Nutzer weiter nach distal bewegt wird, das Gewebe aber nicht in ausreichendem Maß dieser Verschiebung nachgibt.

Ist zusätzlich noch eine zweite Anlagefläche nach lateral vorgesehen, kann der Clip mit den Branchen des Clipapplikators derart zusammenwirken, dass ein aktives Öffnen des Clips über die entspannte, d.h. im Wesentlichen spannungsfreie, Form des Clips hinaus bewirkt werden. Wird das Maul des Clipapplikators mittel Kulissen gesteuert, d.h. geöffnet und geschlossen, so können Kulissen vorgesehen sein, die bei einem Schließvorgang des Mauls des Clipapplikators, also einem Verpressvorgang des Clips, das Maul des Clipapplikators zunächst um einen gewissen Betrag öffnen bevor er anschließend geschlossen wird und damit der Clip verpresst wird. Damit der Clip dieser anfänglichen Öffnungsbewegung folgen kann, ohne aus dem Maul des Clipapplikators zu fallen, und dies auch eine Vergrößerung der lichten Öffnungsweite des Clips bewirkt, gibt es zwei Möglichkeiten. Zum einen kann der Clip elastisch zusammengedrückt werden, bevor er in das Magazin des Clipapplikators eingebracht wird, sodass er in der Ausgangsstellung des Mauls des Clipapplikators elastisch vorgespannt ist. Er kann sich bereits im Magazin des Clipapplikators unter einer elastischen Vorspannung befinden und wird so in das Maul des Clipapplikators eingeführt. Der Clip kann aber auch erst beim Vorschieben in das Maulteil des Clipapplikators elastisch zusammengedrückt und dadurch vorgespannt werden, beispielsweise über einen sich in Querrichtung des Clips verengenden Zuleitungskanal. Wenn sich nun das Maul des Clipapplikators aufweitet, also öffnet, entspannt sich der Clip und die Cliparme folgen jeweils der Öffnungsbewegung der Branchen. Wenn ein Clip aber vorgespannt in ein Magazin eines Clipapplikators eingebracht wird, ist dessen Reibung während des Cliptransports deutlich höher als bei einem entspannten Clip, was wiederum bei der Dimensionierung der mit dem Vorschub befassten Bauteile nachteilig ist. Eine andere Möglichkeit besteht darin, den Clip aktiv aufzuweiten, indem die Branchen seitlich von außen in die Cliparme eingreifen und diese aktiv mit der Öffnungsbewegung des Mauls nach lateral ziehen. In beiden Fällen kann eine Clipöffnung erzielt werden, für die nach dem Stand der Technik ein Applikator mit größerem Durchmesser verwendet werden müsste. Es ist aber insbesondere bei endokopischen Clipapplikatoren immer vorteilhaft, einen Clipapplikator mit kleinerem Durchmesser zu verwenden, da dann kleinere Inzisionen erforderlich sind, was zu einer schnelleren Heilung derselben und zu geringerer Narbenbildung führt. Wenn hier vom Durchmesser eines Clipapplikators gesprochen wird, bezieht sich dies auf den Schaftdurchmesser und nicht auf dessen Griff.

Gemäß wieder einer anderen vorteilhaften Ausführungsform der vorliegenden Erfindung ist die Aussparung in einem distalen Verbindungsbereich zweier distal verbundener Cliparme ausgebildet, vorzugsweise indem die Querschnittsabmessungen des distalen Verbindungsabschnitts nach distal und/oder nach lateral verjüngt sind, wobei die Verjüngung weiter vorzugsweise im Wesentlichen auf die halbe Querschnittsabmessung erfolgt. Wenn diese Aussparung in einem distalen Bereich der Cliparme angeordnet ist, wird eine geringere Kraft zum Aufweiten des Clips benötigt. In diesem Fall müssen die Branchen des Maulteils des Clipapplikators nicht so massiv ausgebildet sein, was für den insgesamt angestrebten schlanken Aufbau des Clipapplikators vorteilhaft ist. Alternativ zu der Bereitstellung einer Aussparung in den Cliparmen können die Anlageflächen auch durch Vorsprünge gebildet werden, die an den Cliparmen vorgesehen sind. Die Aussparung ermöglicht lediglich eine einfachere und kostengünstigere Bereitstellung der jeweiligen Anlageflächen.

Gemäß noch einer weiteren vorteilhaften Ausführungsform der vorliegenden Erfindung weist ein proximaler Verbindungsbereich zweier Cliparme, ein Cliparm und/oder ggf. ein distaler Verbindungsabschnitt zweier Cliparme eine veränderliche Höhe und/oder Breite in wenigstens einer Richtung quer zur neutralen Faser auf, vorzugsweise von mindestens $\pm 10\%$. Da die Verbindungsbereiche der Cliparme nicht wesentlich zur Stabilität der Verpressung beitragen, können diese beispielsweise dünner ausgebildet sein.

Gemäß wieder einer anderen vorteilhaften Ausführungsform der vorliegenden Erfindung vergrößert sich die Querschnittsfläche eines Cliparms und/oder eines proximalen Verbindungsbereichs zu der Clipkehle hin, ohne dass sich die lichte Öffnung am entscheidenden distalen Ende verkleinert, wobei sich insbesondere die Breite des Cliparms und/oder des proximalen Verbindungsbereichs vergrößert. Im Bereich der Clipkehle herrschen im verpressten Zustand des Clips die größten Biegemomente, sodass eine Querschnittsvergrößerung in diesem Bereich die Schließkraft des Clips erhöhen kann, ohne die lichte Öffnung des Clips am distalen Ende zu verringern.

Gemäß noch einer anderen vorteilhaften Ausführungsform der vorliegenden Erfindung weist wenigstens ein Cliparm zumindest einen Abschnitt mit einer Wellenform und/oder einer Zickzackform in einer Richtung parallel und/oder senkrecht zur Clipebene auf. Mit dieser Ausbildung kann die Position des Clips am Gewebe noch besser gesichert werden und ein Abrutschen des Clips vom gegriffenen Gewebe weiter erschwert werden.

Gemäß einer weiteren vorteilhaften Ausführungsform der vorliegenden Erfindung weist ein Paar von Cliparmen von lateral her verjüngte Cliparme auf, vorzugsweise durch einen im distalen Bereich der Cliparme vergrößerten Radius an der Außenkante des Cliparms. Diese Verjüngung der Cliparme muss sich dabei nicht über im Wesentlichen die gesamte Länge des jeweiligen Cliparms erstrecken, sondern kann vorteilhafter Weiser nur in einem bestimmten Bereich vorgesehen sein. Diese Ausbildung ist vorteilhaft, wenn ein Clip in einem extrem klein dimensionierten Schaft eines endoskopischen Clipapplikators untergebracht ist. In diesem Fall kann ein Clipmagazin so beschaffen sein, dass der Clip die Außenwand des Schafts durchdringt, beispielsweise in einem Schlitz, und somit selbst einen Teil der Außenwand des Schafts bildet. Dabei ist es vorteilhaft, wenn der Clip (zumindest der betreffende Bereich des Clips) mit der Mantelfläche des Schafts eine gleichförmige Oberfläche bildet.

Gemäß einer anderen vorteilhaften Ausführungsform der vorliegenden Erfindung weist wenigstens ein Cliparm an seiner Innenfläche ein Profil auf, welches vorzugsweise mittels Prägen, Walzen oder Schneiden ausgebildet ist. Weiter vorzugsweise ist ein atraumatischen Profil vorgesehen. Mit einem solchen Profil kann insbesondere die Position des Clips entlang der Längsachse eines Hohlorgans weiter gesichert werden.

Gemäß wieder einer anderen vorteilhaften Ausführungsform der vorliegenden Erfindung sind die Cliparme eines Paares von Cliparmen und vorzugsweise auch die Cliparme von verschiedenen Paaren von Cliparmen im Wesentlichen gleichförmig ausgebildet. Auf diese Weise muss beim Einbringen der Clips in ein Magazin eines Clipapplikators nicht auf deren Lage geachtet werden. Alternativ können mehrere Paare von Cliparmen eines Clips auch untereinander verschieden sein. Beispielsweise kann bei einem Clip mit drei Paaren von Cliparmen das mittlere Paar von Cliparmen aus zwei gewellten Cliparmen bestehen, wobei die beiden äußeren Paare von Cliparmen aus geraden Cliparmen bestehen. Die Zusammenstellung der einzelnen Paare von Cliparmen kann beliebig erfolgen und auch die Cliparme eines Paares von Cliparmen können gleichartig oder verschiedenartig sein.

Gemäß einem anderen Aspekt der vorliegenden Erfindung weist ein Herstellungsverfahren für einen der vorstehend beschriebenen chirurgischen Clips die folgenden Schritte auf. Stanzen eines Rohlings aus einem Blech, wobei der Rohling an wenigstens einer Stelle mit dem verbleibenden Blech verbunden bleibt, vorzugsweise im Bereich der späteren Clipkehle. Anschließendes Biegen des Rohlings in einer Richtung quer zum verbleibenden Blech, sodass insbesondere die distalen Bereiche der Cliparme von der Blechebene vorstehen und dadurch ein chirurgischer Clip gebildet wird, wobei dieses Biegen in mehreren Schritten erfolgen kann. Zuletzt wird der Clip von dem verbleibenden Blech getrennt. Mit einem solchen Verfahren können die vorstehend beschriebenen Clips kosteneffizient und sicher hergestellt werden. Indem die Clips erst zum Ende des Verfahrens von dem verbleibenden Blech getrennt werden, können diese im Laufe des Produktionsverfahrens nicht verloren gehen und beispielsweise eine Maschine blockieren oder beschädigen. Bei dem vorstehend Beschriebenen Verfahren kann anstelle des Stanzverfahrens auch ein anderes Trennverfahren verwendet werden, wie es beispielsweise vorstehend beschrieben ist, und das Trennen kann mittels Stanzen erfolgen. Die unterschiedliche Benennung dient hier vorwiegend der eindeutigen Identifizierung der einzelnen Verfahrensschritte in der weiteren Beschreibung.

Gemäß einem weiteren Aspekt der vorliegenden Erfindung weist ein alternatives Herstellungsverfahren für einen vorstehend beschriebenen chirurgischen Clip die folgenden Schritte auf. Stanzen eines Rohlings aus einem Blech, wobei der Rohling keine stoffliche Verbindung zu dem verbleibenden Blech beibehält. Anschließendes Klemmen des Rohlings in das verbleibende Blech in dem Bereich, in dem der Rohling aus dem Blech gestanzt wurde. Darauffolgendes Biegen des Rohlings in einer Richtung quer zum verbleibenden Blech, sodass insbesondere die distalen Bereiche der Cliparme von der Blechebene vorstehen und dadurch ein chirurgischer Clip gebildet wird, wobei dieses Biegen in mehreren Schritten erfolgen kann. Zuletzt wird der Clip aus dem verbleibenden Blech heraus genommen. Dieses Verfahren erfordert ein sehr exaktes Ausstanzen des Cliprohlings aus dem Blech. Dafür aber ist keine zweite Stanze o.ä. und kein entsprechender Bearbeitungsschritt mehr erforderlich. Die fertigen Clips können aus dem Blech gedrückt, gerüttelt oder anderweitig heraus genommen werden.

Gemäß einer vorteilhaften Ausführungsform der vorstehend beschriebenen Herstellungsverfahren wird ein Schritt des Prägens eines Profils auf wenigstens eine Seite des Blechs vor und/oder nach dem Stanzen des Rohlings ausgeführt, wobei das Prägen in mehreren Schritten ausgeführt werden kann.

Weitere Vorteile und Merkmale der Erfindung sind dem Fachmann aus den beigefügten Figuren und der detaillierten Beschreibung der Ausführungsbeispiele ersichtlich.

Fig. 1    zeigt eine perspektivische Ansicht eines Doppelstegclips bzw. Ringclips gemäß einem ersten Ausführungsbeispiel;

Fig. 2    zeigt eine Ansicht des Doppelstegclips gemäß Fig. 1 von oben;

Fig. 3    zeigt eine Ansicht des Doppelstegclips gemäß Fig. 1 von der Seite;

Fig. 4    zeigt eine Ansicht des Doppelstegclips gemäß Fig. 1 von vorn (distal);

Fig. 5    zeigt eine Ansicht des Doppelstegclips gemäß Fig. 1 von hinten (proximal);

Fig. 6    zeigt eine Schnittansicht des Doppelstegclips gemäß Fig. 1 durch eine Symmetrieebene des Doppelstegclips;

Fig. 7    zeigt eine Vergrößerung eines Details C aus Fig. 6;

Fig. 8    zeigt eine Ansicht eines Rohlings eines Doppelstegclips von oben nach einem Stanzvorgang und vor einem Biegevorgang und einem Trennvorgang;

Fig. 9    zeigt eine Ansicht des Rohlings gemäß Fig. 8 entlang der Linie A - A;

Fig. 10   zeigt eine Ansicht des Rohlings gemäß Fig. 8 von der Seite nach einem ersten Biegevorgang;

Fig. 11   zeigt eine Ansicht des Rohlings gemäß Fig. 8 von der Seite nach einem zweiten Biegevorgang;

Fig. 12   zeigt eine Ansicht eines Rohlings eines Doppelstegclips von unten nach einem Stanzvorgang gemäß einem zweiten Ausführungsbeispiel;

Fig. 13   zeigt eine Ansicht eines Rohlings eines Doppelstegclips gemäß Fig. 12 von oben nach einem Stanzvorgang und einem Prägevorgang;

Fig. 14   zeigt eine Ansicht des Rohlings gemäß Fig. 13 entlang der Linie B - B;

Fig. 15   zeigt einen Rohling mit veränderlicher Innenkontur;

Fig. 16   zeigt einen Rohling mit veränderlicher Breite über die Länge der Cliparme;

Fig. 17   zeigt einen Rohling mit veränderlicher Höhe über die Länge der Cliparme;

Fig. 18   zeigt einen Clip mit Verpressbuckeln;

Fig. 19   zeigt ein Diagramm, welches die Krümmung eines Clipschenkels über die Länge auf Cliparm gemäß einem Ausführungsbeispiel darstellt; und

Fig. 20   zeigt ein Diagramm, welches den Winkel zwischen Clipschenkel und einer Senkrechten auf die Clipsachse über die Länge auf Cliparm gemäß einem Ausführungsbeispiel darstellt.

Die Ausführungsbeispiele der verschiedenen Aspekte der vorliegenden Erfindung sind im Folgenden unter Bezugnahme auf die Figuren beschrieben.

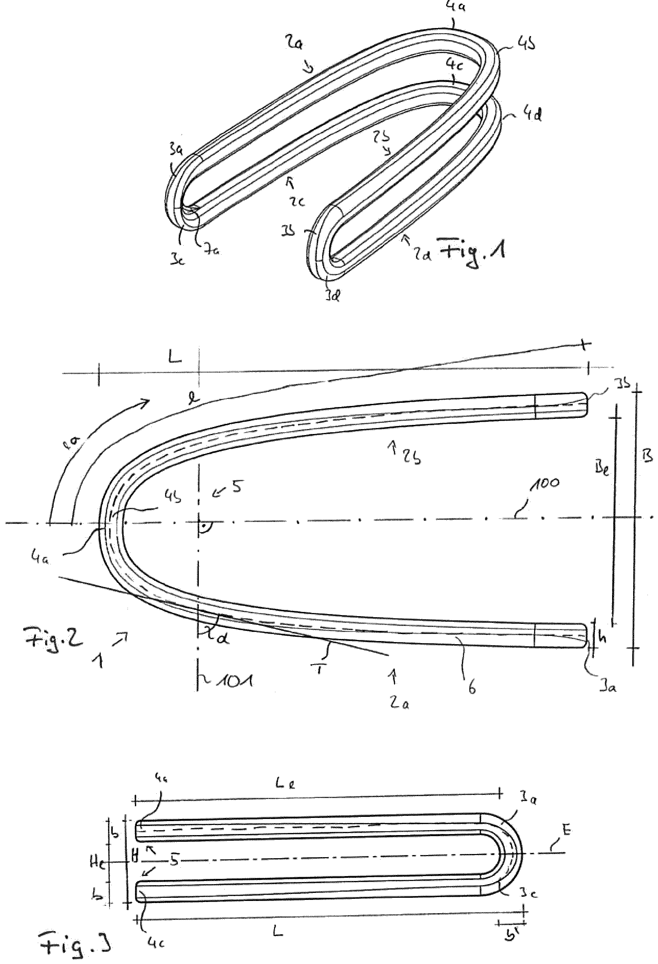

Unter Bezugnahme auf die Fig. 1 bis 7 ist im Folgenden ein erstes Ausführungsbeispiel des ersten Aspekts der vorliegenden Erfindung im Detail beschrieben.

Der in den Fig. 1 bis 7 gezeigte chirurgische Clip 1 ist zur Verwendung mit einem chirurgischen Clipapplikator geeignet, insbesondere mit einem chirurgischen Clipapplikator der Mehrschussart (multi-fire) und weiter vorzugsweise für einen solchen Clipapplikator in Einwegverwendung (sigle use). Der Clip 1 weist zwei Paare von Cliparmen 2a, 2b, 2c, 2d auf, wobei jeder Cliparm 2a, 2b, 2c, 2d ein distales Ende 3a, 3b, 3c, 3d und ein proximales Ende 4a, 4b, 4c, 4d aufweist. Die beiden Cliparme 2a, 2b bzw. 2c, 2d jedes Paares von Cliparmen sind an ihren proximalen Enden 4a, 4b bzw. 4c, 4d miteinander verbunden und bilden so insgesamt zwei Clipkehlen 5 aus. Wie dies in Fig. 2 gezeigt ist, wächst ein Tangentenwinkel a zwischen einer Tangente T an eine neutrale Faser 6 eines Cliparms 2a, 2b, 2c, 2d und einer Senkrechten 101 auf eine Längsachse 100 des Clips 1 über die gesamte Länge l des Cliparms 2a, 2b, 2c, 2d stetig an.

Die Länge des Clips L ist anhand der senkrechten Projektion der Cliparme 2a, 2b, 2c, 2d auf die Längsachse 100 des Clips bestimmt. Die Längsachse 100 des Clips 1 ist bei diesem Ausführungsbeispiel auch eine Schnittlinie zweier sich orthogonal schneidender Symmetrieflächen (Spiegelsymmetrie) des Clips 1. Die Länge l eines Cliparms 2a entspricht der Abwicklung des Cliparms 2a an eine Gerade. Die Position auf einem Cliparm 2a wird durch die Länge $l_a$ dargestellt, wobei $l_a$ im Bereich der Clipkehle (also am proximalen Ende des Clips 1) 0 beträgt und am distalen Ende des Clips 1 l beträgt. Die Höhe h eines Cliparms 2a, 2b, 2c, 2d bestimmt sich aus der Dicke des Rohmaterials, aus dem er zumeist geformt ist. Die Breite b eines Cliparms 2a, 2b, 2c, 2d bestimmt sich daraus, welche Form aus dem Rohmaterial herausgearbeitet wird, das heißt z.B. herausgestanzt wird. Die Höhe H und Breite B des Clips 1 hängt aber nicht von dem Rohmaterial ab, aus dem er gewöhnlich hergestellt wird, sondern wie der aus dem Rohmaterial hergestellte Rohling R geformt wird. daher ergeben sich folgende Zusammenhänge:

1)

$$B = B_l + 2h;$$

2)

$$H = H_l + 2b;$$

wobei $H_l$ die lichte Höhe und $B_l$ die lichte Weite des Clips 1 ist. Zwischen einer bestimmten Position, d.h. bei einer Länge auf Cliparm $l_a$ spannt sich ein Winkel $\alpha$ zwischen einer Tangente an die neutrale Faser 6 eines Cliparms 2a und einer Senkrechte 101 auf die Längsachse 100 des Clips 1 auf. Bei dem vorliegenden Ausführungsbeispiel hat der Tangentenwinkel a bei einer Länge auf Cli-

parm $l_a$ von 6% einen Wert von etwa 25°, bei einer Länge auf Cliparm $l_a$ von 15% einen Wert von etwa 60° und bei einer Länge auf Cliparm $l_a$ von 27% einen Wert von etwa 75°. Zudem hat der Tangentenwinkel a bei einer Länge auf Cliparm $l_a$ von ca. 0% (also in der Clipkehle 5, wo die Cliparme 2a, 2b verbunden sind und in Fig. 2 die Längsachse 100 schneiden) einen Wert von im Wesentlichen 0°. Am distalen Ende des Clips 1, also bei einer Länge auf Cliparm $l_a$ von 100% weist der Tangentenwinkel a einen Wert von etwa 84° auf. Die Abweichungen der Clips von diesen Nennmaßen bewegt sich im Bereich von ± 2°, vorzugsweise von ± 1°.

Die Cliparme 2a, 2b, 2c, 2d des Clips 1 dieses Ausführungsbeispiels sind in der Ebene des Clips 1 von ihrem distalen Ende 3a, 3b, 3c, 3d bis zu ihrem proximalen Ende 4a, 4b, 4c, 4d so geformt, dass eine erste Ableitung der Krümmung der neutralen Faser 6 des Cliparms 2a, 2b, 2c, 2d keinen Vorzeichenwechsel aufweist. Dies bedeutet, dass in dem Cliparm keine Knickstelle vorgesehen ist, an der sich der Tangentenwinkel α im Wesentlichen sprunghaft ändert. Die Cliparme 2a, 2c sind an ihrem distalen Ende 3a, 3c verbunden und bilden einen Verbindungsabschnitt 11 aus und die Cliparme 2b, 2d sind an ihren distalen Enden 3b, 3d verbunden und bilden einen Verbindungsabschnitt 12 aus. Der Clip 1 dieses Ausführungsbeispiels weist zwei Paare von Cliparmen auf und ist als geschlossener Ringclip ausgebildet. Dieser Clip 1 ist aus einem Blech gestanzt, welches auf einer Rolle bezogen werden kann. Üblicherweise werden zwei solche Ringclips nebeneinander aus dem Blech gestanzt.

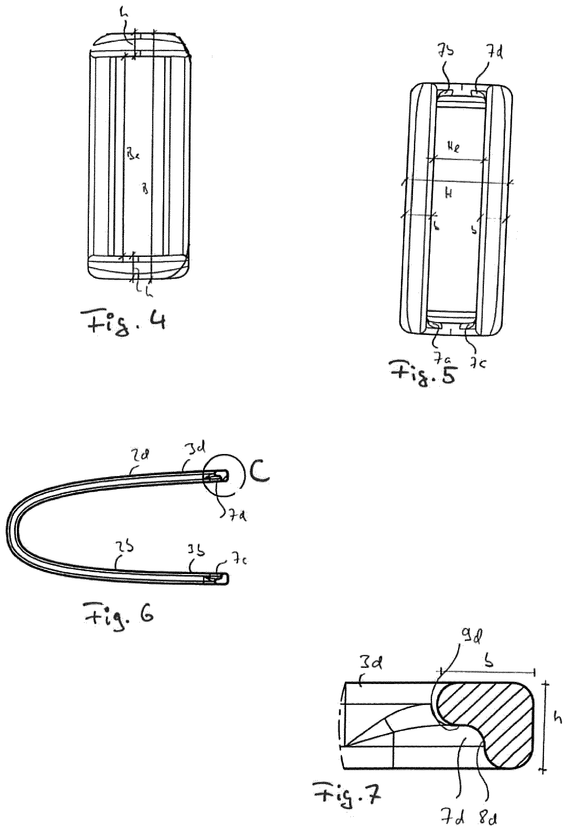

In der Fig. 7 ist ein Schnitt durch einen distalen Bereich 3d des Cliparms 2d im Detail gezeigt. Dort ist eine Aussparung 7d in dem Verbindungsabschnitt 12 ausgebildet, die nach proximal hin offen und nach distal geschlossen ist. Die Aussparung 7d ist dadurch von proximal zugänglich und bildet eine erste Anlagefläche 8d nach distal. Zudem ist die Aussparung 7d von medial her offen und bildet eine zweite Anlagefläche 9d nach lateral. Die Aussparung 7d ist bei diesem Ausführungsbeispiel gleichzeitig mit dem Stanzen ausgebildet. Alternativ dazu kann die Aussparung 7d auch ausgebildet werden, indem das Blech vor dem Ausstanzen des Rohlings R geprägt wird oder indem der Rohling R nach dem Ausstanzen geprägt wird. Dies ist unabhängig davon, nach welchem der unten beschriebenen Herstellungsverfahren hergestellt ist.

Die Aussparung ist ausgebildet, indem die Querschnittsabmessungen b, h des distalen Verbindungsabschnitts 11, 12 nach distal und nach lateral verjüngt sind, wobei die Verjüngung im Wesentlichen auf die halbe Querschnittsabmessung b, h erfolgt.

Bei dem Clip gemäß diesem Ausführungsbeispiel ist sowohl die Höhe h, h' des Blechs und damit der Cliparme 2a, 2b, 2c, 2d als auch deren Breite b, b', b" über die gesamte Länge I der Cliparme 2a, 2bn, 2c, 2d unveränderlich. Zudem weist dieser Clip 1 weder eine Wellenform noch eine Zickzackform in einer Richtung parallel und/oder senkrecht zur Clipebene E auf. Auch weist dieser Clip 1 keine von lateral her verjüngten Cliparme 2c, 2d auf, wie sie vorteilhaft sind, wenn der Clip 1 mit diesen Cliparmen 2c, 2d seitlich aus dem Schaft eines Clipapplikators hervor stehen oder zumindest die Schaftwand durchdringen. Auch weist der Clip 1 dieses Ausführungsbeispiels kein Profil an seinen Cliparmen 2a, 2b, 2c, 2d auf. Der Clip dieses Ausführungsbeispiels ist derart gestaltet, dass eine erste Ableitung der Krümmung einer neutralen Faser des Cliparms 2a, 2b im Bereich einer Länge auf Cliparm $l_a$ von 6,3% einen Vorzeichenwechsel aufweist. Weitere Vorzeichenwechsel zeigt die erste Ableitung der Krümmung der neutralen Faser nicht.

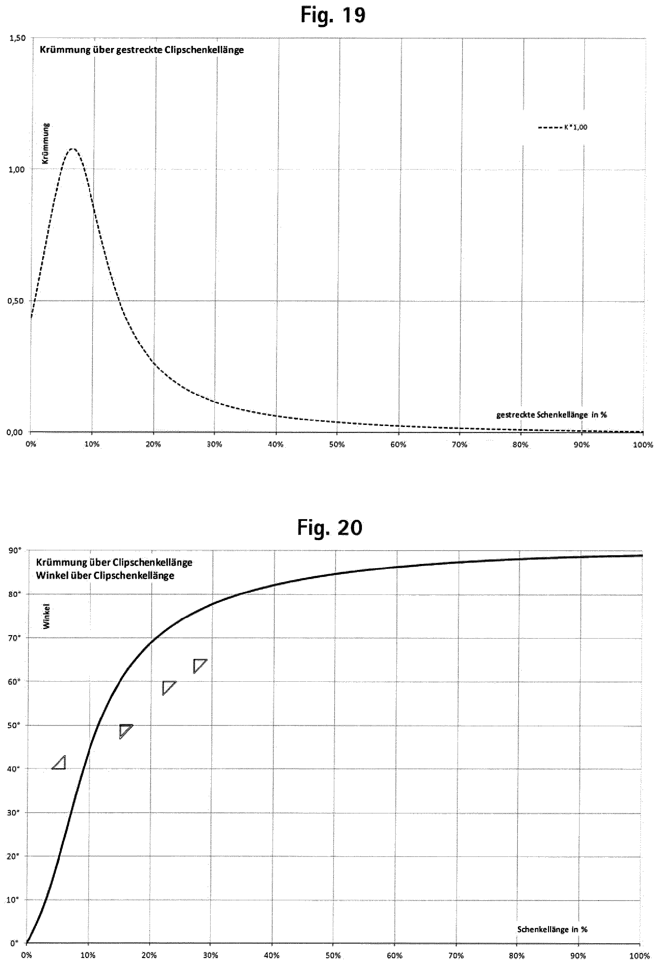

In der Fig. 19 ist der Krümmungsverlauf der neutralen Faser eines Cliparms dieses Clips über die Länge auf dem Cliparm gezeigt. Die Länge auf dem Cliparm ist hierbei relativ zur Cliparmlänge prozentual dargestellt. In der Fig. 20 ist dementsprechend der Winkel dargestellt, den eine Tangente an die neutrale Faser des Clips dieses Ausführungsbeispiels mit einer Geraden einschließt, welche senkrecht auf die Clipachse steht. Auch hier ist die Länge auf dem Cliparm ist hierbei relativ zur Cliparmlänge prozentual dargestellt. Die zusätzlich dargestellten Dreiecke definieren Punkte und somit einen Korridor, in dem sich der Graf bewegen muss, um einen vorteilhaften Clip gemäß dieser Erfindung zu erhalten.

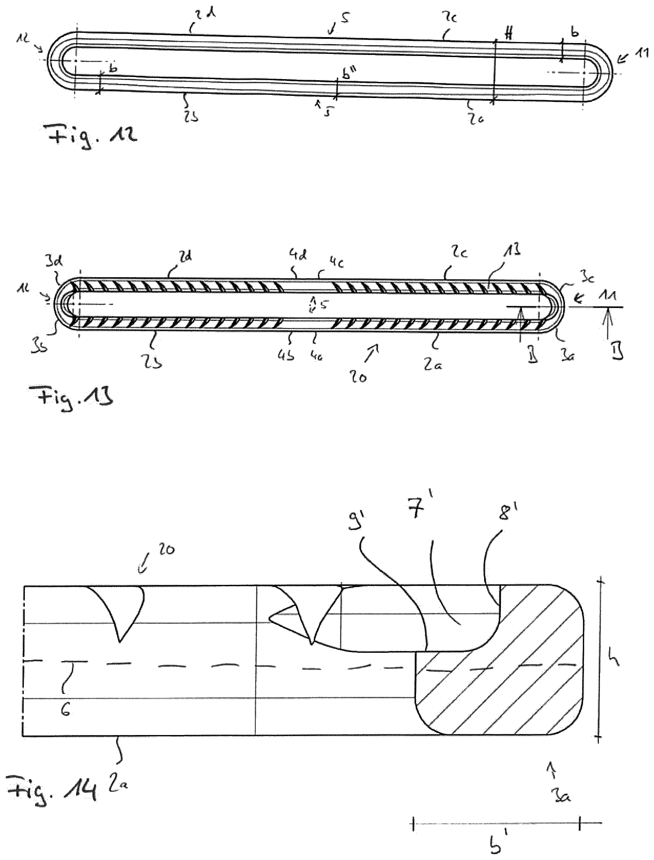

Ein zweites Ausführungsbeispiel des erfindungsgemäßen Clips 1 ist in den Fig. 12 bis 14 gezeigt. Im Folgenden sind nur die Unterschiede zum ersten Ausführungsbeispiel beschrieben. Wie dies insbesondere in Fig. 13 ersichtlich ist, weist der Clip 1 ein Profil an der Innenfläche 13 der Cliparme 2a, 2b, 2c, 2d auf, wobei im Bereich der auszubildenden Clipkehle 5 kein Profil vorgesehen ist. Alternativ dazu kann auch im Bereich der Clipkehle 5 ein Profil vorgesehen sein oder es können an einem oder mehreren Cliparmen 2a, 2b, 2c, 2d Bereiche vorgesehen sein, an denen kein Profil vorgesehen ist. Dieses Profil ist mittels Prägen ausgebildet, wobei das Blech geprägt wurde, bevor der Rohling R aus diesem gestanzt wurde. Im vorliegenden Fall wurde das Profil mit einer Prägewalze auf bzw. in das Blechband auf- bzw. eingebracht. Es ist auch möglich, das Blechband mittels eines Hubstempels zu prägen, die Verwendung einer Prägewalze erleichtert es allerdings, nahtfreie und überschneidungsfreie Prägungen zu erzeugen. Das Profil dieses Clips besteht aus Schlitzförmigen Vertiefungen 20, welche auf einem Cliparm 2a, 2b, 2c, 2d jeweils im Wesentlichen parallel zueinander angeordnet sind. Die Aussparung 7' wurde nicht beim Stanzen des Rohlings R ausgebildet, sondern wurde nach dem Stanzen des Clips 1 gefräst. Auf diese Weise können die Anlageflächen 8', 9' mit schärferen Kanten ausgebildet werden, sodass für Anlageflächen 8', 9', die vergleichbar groß zu den Anlageflächen 8d, 9d des ersten Ausführungsbeispiels sind, die verbleibende Höhe h und Breite b' des Cliparms 2a, 2d größer sein kann. Darüber hinaus ist bei diesem Ausführungsbeispiel die Breite b' der distalen Bereiche 3a, 3b, 3c, 3d der Cliparme 2a, 2b, 2c, 2d geringfügig kleiner als die Breite b der proximalen Bereiche 4a, 4b, 4c, 4d derselben. Insbesondere sind die Verbindungsabschnitte 11, 12 mit einer geringeren Breite b' ausgebildet.

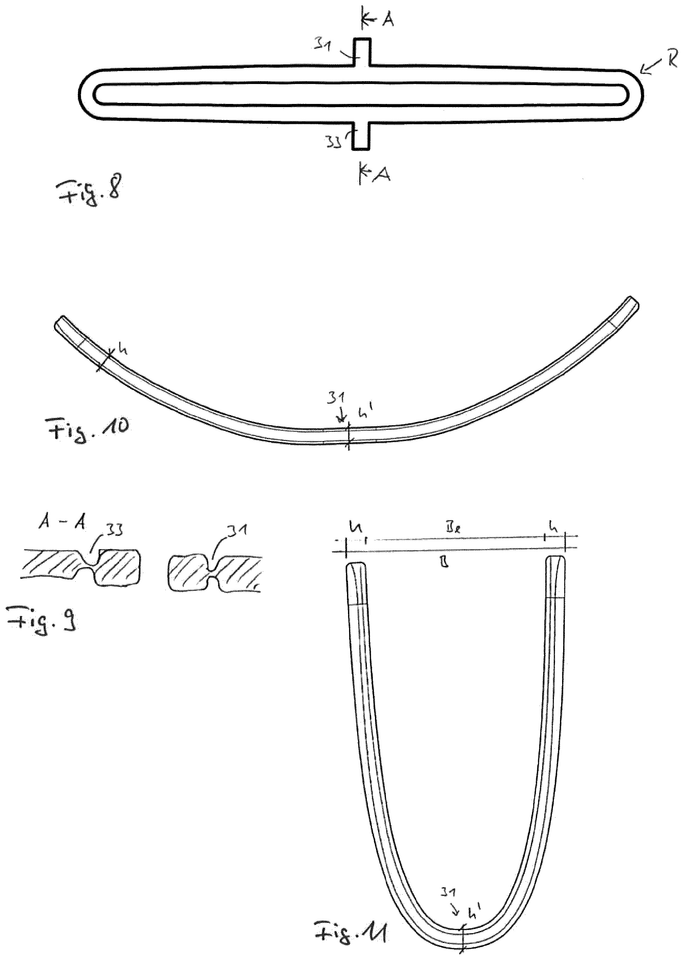

Wie dies vorstehend bereits angedeutet ist, ist der vorliegende Doppelstegclip 1 aus einem Blechband hergestellt, wobei gleichzeitig zwei Clips nebeneinander aus dem Blechband geformt werden. Das Blechband wird zunächst von einer Rolle abgewickelt. In einem ersten Bearbeitungsschritt wird mittels einer Profilwalze ein Profil in die Spätere Innenfläche des Clips 1 eingeprägt. Anschließend wird die grobe Form des späteren Clips 1 aus dem Blech gestanzt und dadurch der Rohling R gebildet. Wie dies in Fig. 8 gezeigt ist, bleibt der Rohling R dabei an zwei Stellen 31, 33 mit dem Blech (nicht gezeigt) verbunden. Allerdings wird die Dicke des Blechs an den Verbindungsstellen 31, 33 stark verringert (siehe Fig. 9), sodass der Clip 1 später leichter von dem verbleibenden Blech getrennt werden kann. Nun wird der Rohling R aus der Blechebene heraus gebogen. Bei diesem Ausführungsbeispiel erfolgt das Biegen in zwei Schritten. Nach einem ersten Biegeschritt weist der Rohling R die in Fig. 10 gezeigte Form auf. Das verbliebende Blech ist in den Fig. 10 und 11 nicht gezeigt. Durch einen zweiten Biegeschritt nimmt der Rohling R die in Fig. 11 gezeigte Form an. Zuletzt werden in einem Trennungsschritt die Verbindungsstellen 31, 33 durchtrennt und so der Rohling R bzw. nun der fertige Clip 1 von dem verbleibenden Blech getrennt. Das Trennen erfolgt durch Stanzen der Verbindungsstellen 31, 33.

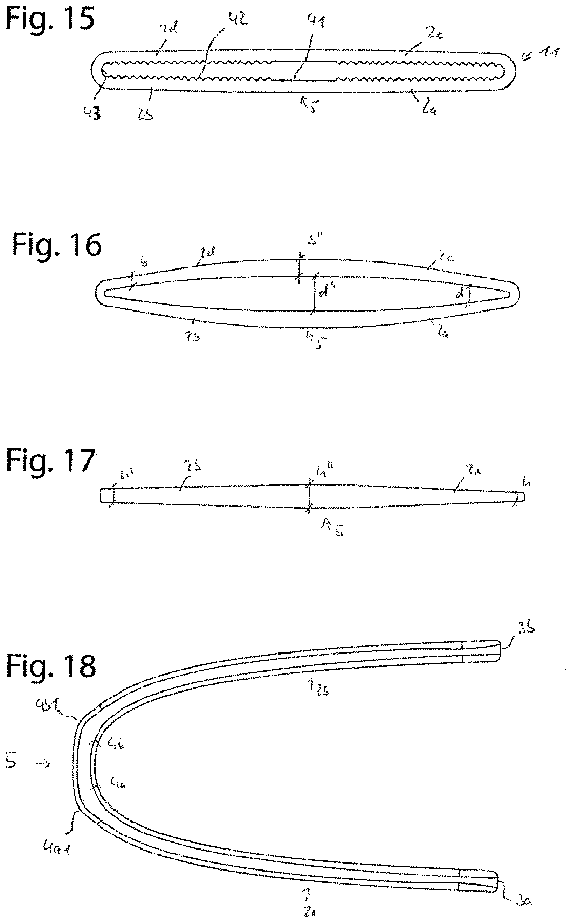

Im Folgenden sind weitere Ausführungsbeispiele und Abwandlungen des Clips beschrieben. Die Fig. 15 zeigt einen Rohling eines Clips entsprechend Fig. 12 und/oder 13 mit veränderlicher Innenkontur 42. Diese wellenförmige Innenkontur 42 der Cliparme 2a, 2b, 2c, 2d sichert den Clip zusätzlich gegen ein Verrutschen des Clips an dem Gefäß in Richtung der Clipachse 100. Die wellenförmige Innenkontur 42 kann auch im Bereich der Clipkehle 5 ausgebildet sein, wobei vorzugsweise in der Clipkehle 5 keine veränderliche Innenkontur 41 vorgesehen ist, um während des Verpressens des Clips keine Spannungsspitzen zu erzeugen, welche ggf. einen Sprödbruch des Materials provozieren könnten. Auch im Bereich der distalen Verbindung zweier Cliparme ist vorzugsweise eine unkonturierte 43 Innenseite der Clipschenkel ausgebildet. Wie dies in Fig. 15 gezeigt ist, können die Wellen der Kontur eine variable Wellenlänge aufweisen. Zudem kann der Übergang von einer nicht gewellt ausgeformten Innenkontur im Bereich der Clipkehle 5 zu einer gewellten Innenkontur mit allmählich zunehmender Wellenamplitude ausgebildet sein. In der Fig. 16 ist ein Rohling eines Clips gezeigt, bei dem die Breite b eines Clipschenkels vom der Clipkehle 5 zu dem distalen Ende des Clipschenkels hin abnimmt. Im Bereich der Clipkehle 5 weist der Clipschenkel die Breite b" auf. Zudem ändert sich auch der Abstand der beiden gegenüberliegenden Clipschenkel 2a, 2c bzw. 2b, 2d von der Clipkehle 5 zu dem distalen Ende des Clips. Im Bereich der Clipkehle 5 weist dieses Ausführungsbeispiel den Abstand d" auf. Die Fig. 17 zeigt einen Rohlings eines Clips gemäß z.B. Fig. 15 oder 16 in einer Seitenansicht. Bei einem Clip dieser Art ist die Höhe des Rohmaterials veränderlich, sodass sich Clips mit veränderlicher Cliparmhöhe h ausbilden lassen. Bei dem vorliegenden Ausführungsbeispiel weist der Cliparm 2a an seinem distalen Ende die Höhe h auf, wohingegen der Cliparm 2b an seinem distalen Ende die Höhe h' aufweist. Im Bereich der Clipkehle 5 weisen beide Cliparme 2a, 2b die Höhe h" auf.

Die Fig. 18 zeigt eine Abwandlung des Clips gemäß Fig. 2, bei dem im Bereich der proximalen Abschnitte 4a, 4b der Cliparme 2a, 2b, also in der Nähe der Clipkehle 5, je ein Verpressbuckel 4a1, 4b1 ausgebildet ist. Diese Verpressbuckel stellen Bereiche der Cliparme dar, welche eine größere Höhe h der Cliparme aufweisen. Diese Verpressbuckel erleichtern das Verpressen des Clips zu einem Zeitpunkt, wenn der Clip schon nahezu vollständig verpresst ist. Genauer gesagt helfen diese Verpressbuckel dabei, das Auge, welches sich beim Verpressen eines solchen Clips im Bereich der Clipkehle 5 ausbildet, klein zu drücken. Im Bereich des Auges kann kein Gewebe fest gehalten werden. Daher ist eine möglichst kleines Clipauge anzustreben.

Die Merkmale der verschiedenen Ausführungsbeispiele sind beliebig geeignet kombinierbar. Darüber hinaus ergeben sich zahlreiche Modifikationen und Abwandlungen im Rahmen der Ansprüche.

So kann der Clip auch mittels Senkerodieren hergestellt werden. Diese Technik kann sowohl anstelle des Fräsens als auch anstelle des Prägens an den entsprechenden Verfahrensschritten eingesetzt werden.

## Patentansprüche

1.  Chirurgischer Clip (1) für einen chirurgischen Clipapplikator mit
    wenigstens einem Paar von Cliparmen (2a, 2b, 2c, 2d), wobei jeder Cliparm (2a, 2b, 2c, 2d) ein distales Ende (3a, 3b, 3c, 3d) und ein proximales Ende (4a, 4b, 4c, 4d) aufweist und die beiden Cliparme (2a, 2b, 2c, 2d) eines Paares von Cliparmen an ihren proximalen Enden (4a, 4b) miteinander verbunden sind, sodass sie eine Clipkehle (5) ausbilden
    ein Tangentenwinkel ($\alpha$) zwischen einer Tangente (T) an eine neutrale Faser (6) eines Cliparms (2a, 2b, 2c, 2d) und einer Senkrechten (101) auf eine Längsachse (100) des Clips (1) über die gesamte Länge (l) des Cliparms (2a, 2b) im Wesentlichen stetig wächst,
    **dadurch gekennzeichnet, dass**
    der Tangentenwinkel ($\alpha$) bei einer Länge auf Cliparm ($l_a$) von 22%, wobei eine Länge auf Cliparm von 0% der Clipkehle entspricht, einen Wert von mindestens 60° aufweist, vorzugsweise einen Wert von mindestens 67° und weiter vorzugsweise einen Wert von 67° bis 80°.

2.  Chirurgischer Clip nach Anspruch 1,
    **dadurch gekennzeichnet, dass**
    der Tangentenwinkel ($\alpha$) bei einer Länge auf Cliparm ($l_a$) von 6% einen Wert von höchstens 40° aufweist, vorzugsweise einen Wert von höchstens 30°, und weiter vorzugsweise einen Wert von 20° bis 30°.

3.  Chirurgischer Clip nach Anspruch 1 oder 2,
    **dadurch gekennzeichnet, dass**
    der Tangentenwinkel ($\alpha$) bei einer Länge auf Cliparm ($l_a$) von 15% einen Wert von mindestens 50° aufweist, vorzugsweise einen Wert von mindestens 58° und weiter vorzugsweise einen Wert von 58° bis 75°.

4.  Chirurgischer Clip nach einem der Ansprüche 1 bis 3,
    **dadurch gekennzeichnet, dass**
    der Tangentenwinkel ($\alpha$) bei einer Länge auf Cliparm ($l_a$) von 27% einen Wert von mindestens 65° aufweist, vorzugsweise einen Wert von mindestens 72°, und weiter vorzugsweise einen Wert von 72° bis 85°.

5.  Chirurgischer Clip nach einem der Ansprüche 1 bis 4,
    **dadurch gekennzeichnet, dass**
    der Tangentenwinkel ($\alpha$) bei einer Länge auf Cliparm ($l_a$) von 100% einen Wert von höchstens 88° aufweist, vorzugsweise einen Wert von 80° bis 88°.

6.  Chirurgischer Clip nach einem der Ansprüche 1 bis 5,
    **dadurch gekennzeichnet, dass**
    erste Ableitung der Krümmung einer neutralen Faser eines Cliparms (2a, 2b) im Bereich einer Länge auf Cliparm von 8% bis 100% keinen Vorzeichenwechsel aufweist, wobei weiter vorzugsweise über die Gesamte Länge des Cliparms kein Vorzeichenwechsel erfolgt.

7.  Chirurgischer Clip nach einem der Ansprüche 1 bis 6,
    **dadurch gekennzeichnet, dass**
    wenigstens zwei Paare von Cliparmen (2a, 2b, 2c, 2d) vorgesehen sind, wobei ein Cliparm (2a, 2b) eines Paares von Cliparmen an seinem distalen Ende (3a, 3b) mit wenigstens einem distalen Ende (3c, 3d) eines anderen Cliparms (2c, 2d) eines anderen Paares von Cliparmen verbunden ist und einen distalen Verbindungsabschnitt (11, 12) bildet.

8.  Chirurgischer Clip nach Anspruch 7,
    **dadurch gekennzeichnet, dass**
    der Clip (1) zwei bzw. drei Paare von Cliparmen aufweist und der Clip (1) als offener oder geschlossener Ringclip oder Doppelringclip ausgebildet ist, vorzugsweise mittels Stanzen, Lasersintern, Walzen, Gießen, Metal Inject Molding und/oder Schneiden, insbesondere Laserschneiden oder Wasserstrahlschneiden.

9.  Chirurgischer Clip nach einem der Ansprüche 1 bis 8,
    **dadurch gekennzeichnet, dass**
    im Bereich des distalen Endes (3a, 3b) eines Cliparms (2a, 2b) und/oder eines distalen Verbindungsabschnitts (11, 12) eine Aussparung (7a, 7b, 7c, 7d, 7') vorgesehen ist, die nach proximal hin offen und nach distal hin geschlossen ist, sodass die Aussparung (7a, 7b, 7c, 7d, 7') von proximal zugänglich ist und eine erste Anlagefläche (8d, 8') nach distal bildet und vorzugsweise eine zweite Anlagefläche (9d, 9') nach lateral oder medial bildet, wobei die Aussparung (7a, 7b, 7c, 7d, 7') weiter vorzugsweise mittels Stanzen und/oder Prägen hergestellt ist.

10. Chirurgischer Clip nach Anspruch 9,
    **dadurch gekennzeichnet, dass**
    die Aussparung (7') in einem distalen Verbindungsbereich (11, 12) zweier distal verbundener Cliparme (2a, 2b; 2c, 2d) ausgebildet ist, vorzugsweise indem die Querschnittsabmessungen (b, b', h, h') des distalen Verbindungsabschnitts (11, 12) auf der proximalen und/oder der medialen Seite des Cliparms ausgebildet ist, wobei eine Reduzierung der Querschnittsabmessung vorzugsweise nach distal und/oder nach lateral erfolgt, wobei weiter vorzugsweise eine Verjüngung im Wesentlichen auf die halbe Querschnittsabmessung (b, b', h, h') erfolgt.

11. Chirurgischer Clip nach einem der Ansprüche 1 bis 10,
    **dadurch gekennzeichnet, dass**
    ein proximaler Verbindungsbereich (5) zweier Cliparme (2a, 2b; 2c, 2d), ein Cliparm (2a, 2b, 2c, 2d) und/oder ggf. ein distaler Verbindungsabschnitt (11, 12) zweier Cliparme (2a, 2b; 2c, 2d) eine veränderliche Höhe (h, h') und/oder Breite (b, b', b'') in wenigstens einer Richtung quer zur neutralen Faser (6) aufweist, vorzugsweise von mindestens ±10%.

12. Chirurgischer Clip nach einem der Ansprüche 1 bis 11,
    **dadurch gekennzeichnet, dass**
    sich die Querschnittfläche eines Cliparms (2a, 2b, 2c, 2b) und/oder eines proximalen Verbindungsbereichs zu der Clipkehle (5) hin verändert, wobei sich insbesondere die Breite (b'') des Cliparms (2a, 2b, 2c, 2b) und/oder des proximalen Verbindungsbereichs verändert, insbesondere vergrößert.

13. Chirurgischer Clip nach einem der Ansprüche 1 bis 12,
    **dadurch gekennzeichnet, dass**

wenigstens ein Cliparm (2a, 2b, 2c, 2b) zumindest einen Abschnitt mit einer Wellenform und/oder einer Zickzackform in einer Richtung parallel und/oder senkrecht zur Clipebene (E) aufweist.

14. Chirurgischer Clip nach einem der Ansprüche 1 bis 13,
   **dadurch gekennzeichnet, dass**
   ein Paar von Cliparmen (2c, 2b) von lateral her im Querschnitt veränderte, insbesondere verjüngte Cliparme (2c, 2b) aufweist, vorzugsweise durch einen Kantenbruch im distalen Bereich (3c, 3d) der Cliparme (2c, 2b), insbesondere einen vergrößerten Radius an der Außenkante des Cliparms (2c, 2b).

15. Chirurgischer Clip nach einem der Ansprüche 1 bis 14,
   **dadurch gekennzeichnet, dass**
   wenigstens ein Cliparm (2a, 2b, 2c, 2d) an seiner Innenfläche (13) ein Profil (20) aufweist, welches vorzugsweise mittels Prägen, Walzen, Schneiden oder mittels Drahterosion oder Senkerosion ausgebildet ist.

16. Herstellungsverfahren für einen chirurgischen Clip nach einem der Ansprüche 1 bis 15,
   **gekennzeichnet durch** die folgenden Schritte:

   Stanzen eines Rohlings (R) aus einem Blech, wobei der Rohling (R) an wenigstens einer Stelle (31, 33) mit dem verbleibenden Blech verbunden bleibt, vorzugsweise im Bereich der späteren Clipkehle (5),
   Biegen des Rohlings (R) in einer Richtung quer zum verbleibenden Blech, sodass die Cliparme (2a, 2b, 2c, 2d) von der Blechebene vorstehen und dadurch ein chirurgischer Clip (1) gebildet wird derart, dass der Tangentenwinkel ($\alpha$) bei einer Länge auf Cliparm ($l_a$) von 22% einen Wert von mindestens 60° aufweist, vorzugsweise einen Wert von mindestens 67° und weiter vorzugsweise einen Wert von 67° bis 80°, wobei dieses Biegen in mehreren Schritten erfolgen kann, und
   Trennen des Clips (1) von dem verbleibenden Blech.

17. Herstellungsverfahren für einen chirurgischen Clip nach einem der Ansprüche 1 bis 15, **gekennzeichnet durch** die folgenden Schritte:

   Stanzen eines Rohlings (R) aus einem Blech, wobei der Rohling (R) keine stoffliche Verbindung zu dem verbleibenden Blech beibehält,
   Klemmen des Rohlings (R) in das verbleibende Blech in dem Bereich, in dem der Rohling (R) aus dem Blech gestanzt wurde,
   Biegen des Rohlings (R) in einer Richtung quer zum verbleibenden Blech, sodass die Cliparme (2a, 2b, 2c, 2d) von der Blechebene vorstehen und dadurch ein chirurgischer Clip (1) gebildet wird, wobei dieses Biegen in mehreren Schritten erfolgen kann, und
   Herausnehmen des Clips (1) aus dem verbleibenden Blech.

18. Herstellungsverfahren für einen chirurgischen Clip nach Anspruch 16 oder 17, wobei ein Schritt des Prägens eines Profils (20) auf wenigstens eine Seite (13) des Blechs vor und/oder nach dem Stanzen des Rohlings (R) ausgeführt wird, wobei das Prägen in mehreren Schritten ausgeführt werden kann.

## Claims

1. A surgical clip (1) for a surgical clip applicator, having

   at least one pair of clip arms (2a, 2b, 2c, 2d), wherein each clip arm (2a, 2b, 2c, 2d) has a distal end (3a, 3b, 3c, 3d) and a proximal end (4a, 4b, 4c, 4d) and the two clip arms (2a, 2b, 2c, 2d) of a pair of clip arms are connected to each other at their proximal ends (4a, 4b) to form a clip throat (5),
   wherein
   a tangent angle ($\alpha$) between a tangent (T) to a neutral fiber (6) of a clip arm (2a, 2b, 2c, 2d) and a perpendicular (101) to a longitudinal axis (100) of the clip (1) increases over the entire length (1) of the clip arm (2a, 2b) substantially continuously
   **characterized in that**
   the tangent angle ($\alpha$) has, on a length on the clip arm ($l_a$) of 22%, wherein a length on the clip arm of 0% corresponds to the clip throat, a value of at least 60°, preferably a value of at least 67°, and further preferably a value of 67° to 80°.

2. A surgical clip as claimed in claim 1,

   **characterized in that**
   the tangent angle ($\alpha$) has, on a length on the clip arm ($l_a$) of 6%, a value not exceeding 40°, preferably a value not exceeding 30°, and further preferably a value not exceeding 20° to 30°.

3. A surgical clip as claimed in claim 1 or 2,

   **characterized in that**
   the tangent angle ($\alpha$) has, on a length on the clip arm ($l_a$) of 15%, a value of at least 50°, preferably a value of at least 58°, and further preferably a value of 58° to 75°.

4. A surgical clip as claimed in one of claims 1 to 3,

**characterized in that**
the tangent angle ($\alpha$) has, on a length on the clip arm ($l_a$) of 27%, a value of at least 65°, preferably a value of at least 72°, and further preferably a value of 72° to 85°.

5. A surgical clip as claimed in one of claims 1 to 4,

   **characterized in that**
   the tangent angle ($\alpha$) has, on a length on the clip arm ($l_a$) of 100%, a value not exceeding 88°, and preferably a value of 80° to 88°.

6. A surgical clip as claimed in one of claims 1 to 5,

   **characterized in that**
   a first derivation of the curvature of a neutral fiber of a clip arm (2a, 2b) has no change of sign in the region of a length on the clip arm of 8% to 100%, wherein further preferably no change of sign occurs over the entire length of the clip arm.

7. A surgical clip as claimed in one of claims 1 to 6,

   **characterized in that**
   at least two pairs of clip arms (2a, 2b, 2c, 2d) are provided, wherein a clip arm (2a, 2b) of one pair of clip arms is at its distal end (3a, 3b) connected with at least a distal end (3c, 3d) of another clip arm (2c, 2d) of another pair of clip arms, and forms a distal connection portion (11, 12).

8. A surgical clip as claimed in claim 7,

   **characterized in that**
   the clip (1) has two and three, respectively, pairs of clip arms, and the clip (1) is formed into an open or closed ring clip or double ring clip, preferably by means of stamping, laser sintering, rolling, casting, metal inject molding and/or cutting, in particular laser cutting or water jet cutting.

9. A surgical clip as claimed in one of claims 1 to 8,

   **characterized in that**
   a recess (7a, 7b, 7c, 7d, 7') is provided in the region of the distal end (3a, 3b) of a clip arm (2a, 2b) and/or a distal connection portion (11, 12) which is open in the proximal direction and closed in the distal direction so that the recess (7a, 7b, 7c, 7d, 7') is proximally accessible and forms a first abutment face (8d, 8') in the distal direction and preferably forms a second abutment face (9d, 9') in the lateral direction or the medial direction, wherein the recess (7a, 7b, 7c, 7d, 7') is further preferably made by means of stamping and/or punching.

10. A surgical clip as claimed in claim 9,

    **characterized in that**
    the recess (7') is formed in a distal connection region (11, 12) of two distally connected clip arms (2a, 2b; 2c, 2d), preferably by forming the sectional dimensions (b, b', h, h') of the distal connection region (11, 12) on the proximal and/or medial side of the clip arm, wherein a reduction of the sectional dimension is effected preferably in the distal and/or the lateral direction, wherein further preferably a tapering is effected to substantially half the sectional dimension (b, b', h, h').

11. A surgical clip as claimed in one of claims 1 to 10,

    **characterized in that**
    a proximal connection region (5) of two clip arms (2a, 2b; 2c, 2d), one clip arm (2a, 2b, 2c, 2d) and/or if need be a distal connection portion (11, 12) of two clip arms (2a, 2b; 2c, 2d) has a variable height (h, h') and/or width (b, b', b") in at least one direction perpendicular to the neutral fiber (6), preferably of at least ±10%.

12. A surgical clip as claimed in one of claims 1 to 11,

    **characterized in that**
    the sectional area of a clip arm (2a, 2b, 2c, 2b) and/or a proximal connection region vary toward the clip throat (5), wherein in particular the width (b") of the clip arm (2a, 2b, 2c, 2b) and/or the proximal connection region varies, and in particular increases.

13. A surgical clip as claimed in one of claims 1 to 12,

    **characterized in that**
    at least one clip arm (2a, 2b, 2c, 2b) has at least a portion having a wave form and/or a zigzag form in a direction parallel and/or perpendicular to the clip plane (E).

14. A surgical clip as claimed in one of claims 1 to 13,

    **characterized in that**
    a pair of clip arms (2c, 2b) has clip arms (2c, 2b) that are varied in cross-section, in particular tapered, from a lateral direction, preferably by means of a chamfer in the distal region (3c, 3d) of the clip arms (2c, 2b), and in particular an enlarged radius at the outer edge of the clip arm (2c, 2b).

15. A surgical clip as claimed in one of claims 1 to 14,

**characterized in that**
at least one clip arm (2a, 2b, 2c, 2d) has a profile (20) at its inside area (13) which is formed preferably by punching, rolling, cutting or by means of wire-electro discharge machining or ram electrical discharge machining.

16. A method of manufacturing a surgical clip as claimed in one of claims 1 to 15,
    **characterized by** the following steps:

    stamping a blank (R) out of a sheet metal, wherein the blank (R) is kept connected to the remaining sheet metal at at least one position (31, 33), preferably in the region of the future clip throat (5),
    bending the blank (R) in a direction perpendicular to the remaining sheet metal so that the clip arms (2a, 2b, 2c, 2d) project from the sheet metal plane and a surgical clip (1) is formed thereby such that the tangent angle ($\alpha$) has, on a length on the clip arm ($l_a$) of 22%, a value of at least 60°, preferably a value of at least 67°, and further preferably a value of 67° to 80°, wherein such bending can be effected in plural steps, and separating the clip (1) from the remaining sheet metal.

17. A method of manufacturing a surgical clip according to one of claims 1 to 15,
    **characterized by** the following steps:

    stamping a blank (R) out of a sheet metal, wherein the blank (R) does not maintain any material connection to the remaining sheet metal,
    clamping the blank (R) into the remaining sheet metal in the region in which the blank (R) has been stamped out of the sheet metal,
    bending the blank (R) in a direction perpendicular to the remaining sheet metal so that the clip arms (2a, 2b, 2c, 2d) project from the sheet metal plane and a surgical clip (1) is formed thereby, wherein such bending can be effected in plural steps, and
    taking out the clip (1) of the remaining sheet metal.

18. A manufacturing method for a surgical clip as claimed in claim 16 or 17, wherein a step of punching of a profile (20) is carried out on at least one side (13) of the sheet metal prior to and/or after the stamping of the blank (R), wherein the punching can be performed in plural steps.

**Revendications**

1. Clamp chirurgical (1) pour un applicateur de clamp chirurgical avec
   au moins une paire de bras de clamp (2a, 2b, 2c, 2d), dans lequel chaque bras de clamp (2a, 2b, 2c, 2d) présente une extrémité distale (3a, 3b, 3c, 3d) et une extrémité proximale (4a, 4b, 4c, 4d) et les deux bras de clamp (2a, 2b, 2c, 2d) d'une paire de bras de clamp sont reliés l'un à l'autre par leur extrémité proximale (4a, 4b), de sorte qu'ils forment une gorge de clamp (5)
   un angle tangentiel ($\alpha$) croît essentiellement en continu entre une tangente (T) sur une fibre neutre (6) d'un bras de clamp (2a, 2b, 2c, 2d) et une perpendiculaire (101) à un axe longitudinal (100) du clamp (1) sur toute la longueur (l) du bras de clamp (2a, 2b),
   **caractérisé en ce que**
   l'angle tangentiel ($\alpha$), pour une longueur sur le bras de clamp ($l_a$) de 22 %, dans lequel une longueur sur le bras de clamp de 0 % correspond à la gorge de clamp, présente une valeur d'au moins 60°, de préférence une valeur d'au moins 67° et mieux encore une valeur de 67° à 80°.

2. Clamp chirurgical selon la revendication 1,
   **caractérisé en ce que**
   l'angle tangentiel ($\alpha$), pour une longueur sur le bras de clamp ($l_a$) de 6 %, présente une valeur d'au plus 40°, de préférence une valeur d'au plus 30°, et mieux encore une valeur de 20° à 30°.

3. Clamp chirurgical (1) selon la revendication 1 ou 2,
   **caractérisé en ce que**
   l'angle tangentiel ($\alpha$), pour une longueur sur le bras de clamp ($l_a$) de 15 %, présente une valeur d'au plus 50°, de préférence une valeur d'au moins 58°, et mieux encore une valeur de 58° à 75°.

4. Clamp chirurgical selon l'une quelconque des revendications 1 à 3,
   **caractérisé en ce que**
   l'angle tangentiel ($\alpha$), pour une longueur sur le bras de clamp ($l_a$) de 27 %, présente une valeur d'au moins 65°, de préférence une valeur d'au moins 72°, et mieux encore une valeur de 72° à 85°.

5. Clamp chirurgical selon l'une quelconque des revendications 1 à 4,
   **caractérisé en ce que**
   l'angle tangentiel ($\alpha$), pour une longueur sur le bras de clamp ($l_a$) de 100 %, présente une valeur d'au plus 88°, de préférence une valeur de 80° à 88°.

6. Clamp chirurgical selon l'une quelconque des revendications 1 à 5,
   **caractérisé en ce que**
   première dérivation de la courbure d'une fibre neutre d'un bras de clamp (2a, 2b) dans la plage d'une longueur de bras de clamp de 8 % à 100 % ne présente aucun changement de signe, dans lequel mieux encore aucun changement de signe n'a lieu sur toute la longueur du bras de clamp.

7. Clamp chirurgical selon l'une quelconque des revendications 1 à 6,
   **caractérisé en ce que**
   au moins deux paires de bras de clamp (2a, 2b, 2c, 2d) sont prévues, dans lequel un bras de clamp (2a, 2b) d'une paire de bras de clamp est relié à son extrémité distale (3a, 3b) à au moins une extrémité distale (3c, 3d) d'un autre bras de clamp (2c, 2d) d'une autre paire de bras de clamp et forme une partie de liaison distale (11, 12).

8. Clamp chirurgical selon la revendication 7,
   **caractérisé en ce que**
   le clamp (1) présente deux ou trois paires de bras de clamp et le clamp (1) est réalisé sous la forme de clamp à anneau ou clamp à double anneau ouvert ou fermé, de préférence par estampage, frittage laser, laminage, coulée, moulage par injection de poudre et/ou découpage, en particulier découpage au laser ou découpage au jet d'eau.

9. Clamp chirurgical selon l'une quelconque des revendications 1 à 8,
   **caractérisé en ce que**
   un évidement (7a, 7b, 7c, 7d, 7') est prévu dans la zone de l'extrémité distale (3a, 3b) d'un bras de clamp (2a, 2b) et/ou d'une partie de liaison distale (11, 12), qui est ouvert en direction du côté proximal et en direction du côté distal, de sorte que l'évidement (7a, 7b, 7c, 7d, 7') est accessible depuis le côté proximal et forme vers le côté distal une première surface d'appui (8d, 8') et forme de préférence une deuxième surface d'appui (9d, 9') vers le côté latéral ou médial, dans lequel l'évidement (7a, 7b, 7c, 7d, 7') est produit en outre de préférence par estampage et/ou gaufrage.

10. Clamp chirurgical selon la revendication 9,
    **caractérisé en ce que**
    l'évidement (7') est formé dans une zone de liaison distale (11, 12) de deux bras de clamp (2a, 2b; 2c, 2d) reliés du côté distal, de préférence du fait que les dimensions de section transversale (b, b', h, h') de la partie de liaison distale (11, 12) est formée sur le côté proximal et/ou le côté médial du bras de clamp, dans lequel une réduction de la dimension de section transversale s'effectue vers le côté distal et/ou vers le côté latéral, dans lequel mieux encore un amincissement s'effectue essentiellement sur la demi-dimension de section transversale (b, b', h, h').

11. Clamp chirurgical selon l'une quelconque des revendications 1 à 10,
    **caractérisé en ce que**
    une zone de liaison proximale (5) de deux bras de clamp (2a, 2b; 2c, 2d), un bras de clamp (2a, 2b, 2c, 2d) et/ou le cas échéant une partie de liaison distale (11, 12) de deux bras de clamp (2a, 2b; 2c, 2d) présente une hauteur (h, h') et/ou largeur (b, b', b'') variable dans au moins une direction transversale à la fibre neutre (6), de préférence d'au moins $\pm$ 10 %.

12. Clamp chirurgical selon l'une quelconque des revendications 1 à 11,
    **caractérisé en ce que**
    la surface de section transversale d'un bras de clamp (2a, 2b, 2c, 2b) et/ou d'une zone de liaison proximale change en direction de la gorge de clamp (5), dans lequel la largeur (b'') du bras de clamp (2a, 2b, 2c, 2b) et/ou de la zone de liaison proximale change, en particulier augmente.

13. Clamp chirurgical selon l'une quelconque des revendications 1 à 12,
    **caractérisé en ce que**
    au moins un bras de clamp (2a, 2b, 2c, 2b) présente au moins une partie avec une forme ondulée et/ou une forme en zigzag dans une direction parallèle et/ou perpendiculaire au plan de clamp (E).

14. Clamp chirurgical selon l'une quelconque des revendications 1 à 13,
    **caractérisé en ce que**
    une paire de bras de clamp (2c, 2b) présente des bras de clamp (2c, 2b) modifiés en section transversale, en particulier amincis, depuis le côté latéral, de préférence au moyen d'un chanfrein dans la zone distale (3c, 3d) des bras de clamp (2c, 2b), en particulier d'un rayon agrandi sur le bord extérieur du bras de clamp (2c, 2b).

15. Clamp chirurgical selon l'une quelconque des revendications 1 à 14,
    **caractérisé en ce que**
    au moins un bras de clamp (2a, 2b, 2c, 2d) présente sur sa surface intérieure (13) un profilé (20), lequel est réalisé de préférence par estampage, laminage, découpage ou par découpage par électroérosion à l'aide d'un fil ou enfonçage par électroérosion.

16. Procédé de fabrication pour un clamp chirurgical selon l'une quelconque des revendications 1 à 15,
    **caractérisé par** les étapes suivantes :

    l'estampage d'une ébauche (R) à partir d'une tôle, dans lequel l'ébauche (R) reste reliée à la tôle résiduelle en au moins un endroit (31, 33), de préférence dans la zone de la gorge de clamp (5) ultérieure,
    le pliage de l'ébauche (R) dans une direction transversale à la tôle résiduelle, de sorte que les bras de clamp (2a, 2b, 2c, 2d) font saillie du plan de tôle et un clamp chirurgical (1) est formé de cette façon, de telle sorte que l'angle tangentiel (a) présente pour une longueur sur le bras de clamp (l$_a$) de 22 % une valeur d'au moins 60°, de préférence une valeur d'au moins 67° et mieux encore une valeur de 67° à 80°, dans lequel ce pliage peut s'effectuer en plusieurs étapes, et

la séparation du clamp (1) de la zone résiduelle.

17. Procédé de fabrication pour un clamp chirurgical selon l'une quelconque des revendications 1 à 15, **caractérisé par** les étapes suivantes :

l'estampage d'une ébauche (R) à partir d'une tôle, dans lequel l'ébauche (R) ne conserve aucune liaison matérielle par rapport à la tôle résiduelle,
le serrage de l'ébauche (R) dans la tôle résiduelle dans la zone dans laquelle l'ébauche (R) a été estampée de la tôle,
le pliage de l'ébauche (R) dans une direction transversale à la tôle résiduelle, de sorte que les bras de clamp (2a, 2b, 2c, 2d) font saillie du plan de tôle et un clamp chirurgical (1) est formé de cette façon, dans lequel ce pliage peut s'effectuer en plusieurs étapes, et
le retrait du clamp (1) de la zone résiduelle.

18. Procédé de fabrication pour un clamp chirurgical selon la revendication 16 ou 17, dans lequel une étape de l'estampage d'un profilé (20) sur au moins un côté (13) de la tôle est réalisée avant et/ou après l'estampage de l'ébauche (R), dans lequel l'estampage peut être réalisé en plusieurs étapes.

Fig. 1

Fig. 2

Fig. 3

Fig. 4

Fig. 5

Fig. 6

Fig. 7

Fig. 8

Fig. 10

A – A    Fig. 9

Fig. 11

Fig. 12

Fig. 13

Fig. 14

Fig. 15

Fig. 16

Fig. 17

Fig. 18

# Fig. 19

# Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2389878 A1
- EP 1810662 A1
- EP 0576835 A2
- US 20110224701 A1